# EUROPEAN PATENT APPLICATION

(11) **EP 3 831 410 A1**
(43) Date of publication of application: **09.06.2021**
(21) Application number: 19830722.5
(22) Date of filing: 10.04.2019
(51) Int. Cl.: A61K 45/06, A61K 39/395, A61P 35/00

(54) **PHARMACEUTICAL COMPOSITION FOR TREATING B CELL LYBPHOMA**

(30) Priority: 04.07.2018 CN 201810723884
(71) Applicant: Fudan University Shanghai Cancer Center, Xuhui, Shanghai 200032 (CN)
(72) Inventor: HU, Weiguo, Shanghai 200032 (CN); CHEN, Jianfeng, Shanghai 200032 (CN)
(74) Representative: Ipside
(86) International application number: PCT/CN2019/082103
(87) International publication number: WO 2020/007087

(57) **Abstract**

The present invention discloses a pharmaceutical composition for treating B-cell lymphoma, including a PI3K/AKT signaling pathway inhibitor and a chemotherapeutic drug, and further including a monoclonal antibody targeting CD20, such as rituximab. The pharmaceutical composition provided by the present invention is particularly suitable for the treatment of relapsed/refractory B-cell lymphoma. Given the critical role of cancer stem cells (CSCs) in the process of metastasis and drug resistance, the present invention proposes a new pro-differentiation therapy (PDT) strategy to cope with CSCs, i.e. to determine the decisive signaling pathway that maintains stem cell sternness and then promotes CSC differentiation by interfering with the pathway. Differentiated cells are eventually sensitive to conventional therapies, such as chemotherapy. In this situation, the PI3K/AKT signaling pathway inhibitor in combination with an R-CHOP regimen has a good effect on the treatment of drug-resistant diffuse large B-Cell lymphoma (DLBCL).

## Description

### Technical Field

The present invention relates to a pharmaceutical composition for treating B-cell lymphoma, in particular to a pharmaceutical composition for treating relapsed/refractory B-cell lymphoma.

### Background of the Invention

The problem of drug resistance has always been a major problem in cancer therapy, and such acquired drug resistance derives, at least in part, from cell type heterogeneity within tumors, including various rapidly growing differentiated cancer cells and very few slowly growing cancerstem cells (CSCs). Although traditional tumor therapies, such as radiotherapy and chemotherapy, can eliminate most of the differentiated cells in the tumor, cancer stem cells still survive, which may be an important reason for promoting the development of drug resistance. CSCs were initially found in leukemias in a very low proportion, but new tumors can be seeded, which is subsequently further confirmed in solid tumors. CSCs have specific membrane protein markers, self-renewal, differentiation, and resting capacity, as well as features highly associated with drug resistance, recurrence, and metastasis. Accordingly, those skilled in the art have been devoted to developing a combination therapy of traditional therapy and CSC-specific therapy.

A series of genetic events accumulated for several years or decades, including activation or overexpression of oncogenes, and/or underexpression or lack of expression of cancer suppressor genes, can ultimately lead to the occurrence and development of tumors. As the tumor progresses, the tumor gradually loses the differentiated phenotype and acquires stem cell-like characteristics, the proportion of CSCs increases, leading to tumor distant metastases, and drug resistance to treatment. However, various attempts to reverse drug resistance by indirectly slowing down the growth of CSCs or directly clearing CSCs have been far from successful, including CSC-dependent signaling molecules (Wnt/β-catenin, Hedgehog, Notch, NF-B, FAK, PTEN, Nanog, JAK/STAT, etc.) inhibitors, tumor microenvironment modulators, CD44-targeted nanoparticle drugs or CD133-targeted immunotherapy, and differentiation therapy. It is worth noting that differentiation therapy to treat acute promyelocytic leukemia (APL) with all-trans retinoic acid (ATRA)/arsenic trioxide (ATO) drugs is not directed at CSCs. APL is characterized by expression of a PML/RARA fusion gene product, whereas application of ATRA alone achieves a complete remission rate of about 85% in APL patients by inducing degradation, apoptosis and terminal differentiation of PML/RARa. However, this differentiation therapy is ineffective against other hematopoietic malignancies and solid tumors, especially their CSCs. Currently, differentiation therapy protocols that specifically target CSCs primarily employ histone deacetylase inhibitors (such as suberoylanilide hydroxamic acid) and histone methyltransferase inhibitors (such as EZH2 inhibitors), however, these epigenetic drugs are still in the early stages of research and their side effects still need to be further evaluated.

SOX2 is a cellular sternness-associated transcription factor that is crucial for sternness maintenance in embryonic stem cells and induced pluripotent stem cells. However, the expression of SOX2 in tumor cells can promote tumor proliferation, invasion and metastasis, and induce drug resistance to therapy, which has a very negative impact on tumor treatment. Studies on SOX2 expression regulation have focused primarily on the regulation of SOX2 by non-coding RNAs, while there are few reports on transcriptional regulation and post-translational modification of SOX2. In addition, the PI3K/AKT signaling pathway not only plays an important regulatory role in tumorigenesis, tumor progression, and drug resistance, but also plays an important role in the regulation of CSCs. It has been reported that activation of the PI3K/AKT signaling pathway can inhibit the ubiquitination of SOX2 by regulating the methylation (K119) and phosphorylation (T118) switches of SOX2, thereby increasing the protein stability of SOX2.

The mortality rate of tumor patients caused by non-Hodgkin's lymphoma (NHL) has ranked among the top ten tumor-related mortality rates, with diffuse large B-cell lymphoma (DLBCL) being the most common subtype of NHL, accounting for 30% of all adult lymphomas. According to the results of gene expression profile analysis, DLBCL is currently further subdivided into three different subtypes: germinal center B-cell-like (GCB), activated B-cell-like (ABC), and primary mediastinal B-cell lymphoma (PMBL) in a lower proportion (10-20%). Although half or more of DLBLC patients can be cured by R-CHOP (rituximab/R, cyclophosphamide/C, doxorubicin/H, vincristine/O, and prednisone/P) regimens, as many as one third of patients eventually relapse. The remaining alternative treatment regimens for these patients, including high-dose chemotherapy and autologous stem cell transplantation, are extremely limited in effect, with a very low success rate, and ultimately lead to death. Therefore, there is an urgent need to develop a new method for treating relapsed/refractory DLBCL.

### Summary of the Invention

In view of the above problems existing in the prior art, it is an object of the present invention to provide a method and drug for treating B-cell lymphoma, in particular a method and drug for treating relapsed/refractory B-cell lymphoma.

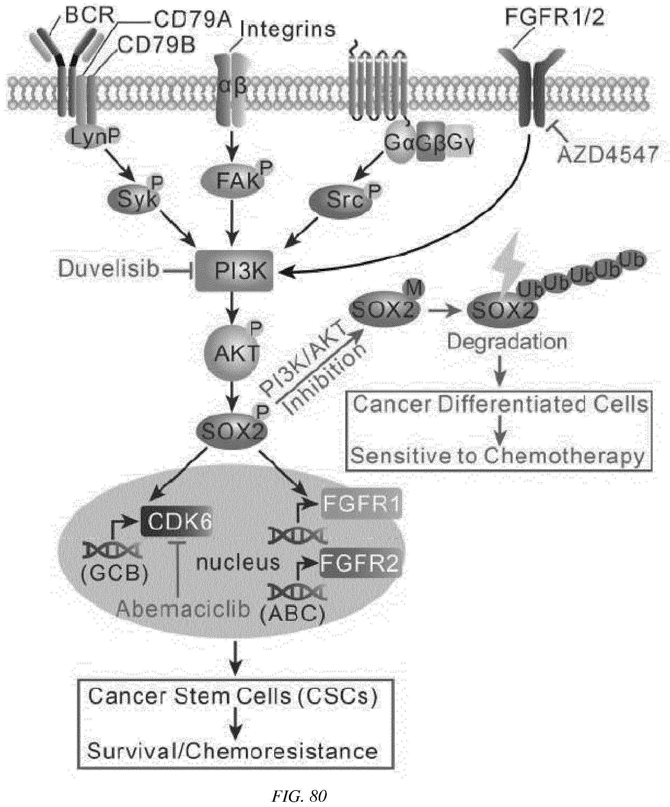

To achieve the above purpose, the present invention has found a unique treatment strategy for CSCs through extensive experimental studies, called pro-differentiation therapy (PDT). The treatment mechanism of this method is shown in Fig. 80. Briefly, in RCHO-resistant DLBCL cells, BCR-and TCF-3/SHP-1-mediated Syk, integrin, CCR7 and FGFR1/2 signaling molecules can all activate the PI3K/AKT pathway, and then enhance SOX2 phosphorylation and reducing SOX2 methylation, thereby stabilizing SOX2 from being degraded by ubiquitination, and finally, up-regulated SOX2 can increase the proportion of cancer stem cells (CSCs) through CDK6 or FGFR1/2 (depending on the subtype of drug-resistant DLBCL cells), and prolong the survival time of CSCs, thereby inducing drug resistance of DLBCL cells to RCHO. When combined with R-CHOP, PI3K/AKT signaling pathway inhibitors promote CSC differentiation by accelerating ubiquitinated degradation of SOX2, resulting in originaldrug-resistant DLBCL cells being aberrantly sensitive to R-CHOP treatment, ultimately completely reversing drug resistance.

Based on the above treatment principle, the present invention first provides a pharmaceutical composition for treating B-cell lymphoma, including a drug promoting CSC differentiation and a chemotherapeutic drug.

Further, the pharmaceutical composition for treating B-cell lymphoma includes a PI3K/AKT signaling pathway inhibitor and a chemotherapeutic drug.

Further, the chemotherapeutic drug may be any one or more chemotherapeutic drugs effective against the tumor. Preferably the chemotherapeutic drug is selected from one or more of cyclophosphamide (C), doxorubicin (H) and vincristine (O); in a preferred embodiment of the present invention, the chemotherapeutic drug is a combination of the aforementioned three drugs and a hormonal drug prednisone (P).

Preferably, the pharmaceutical composition for treating B-cell lymphoma further includes a monoclonal antibody targeting CD20, including but not limited to rituximab (R).

Further, the pharmaceutical composition for treating B-cell lymphoma further includes a pharmaceutically acceptable carrier or excipient.

Further, the PI3K/AKT signaling pathway inhibitor may be selected from PI3K inhibitors and AKT inhibitors; still further, the PI3K/AKT signaling pathway inhibitor may be an inhibitor of related proteins upstream and downstream of PI3K/AKT.

Further, the PI3K inhibitors are broad-spectrum PI3K inhibitors or subtype-specific PI3K inhibitors; The PI3K inhibitors include, but are not limited to, thienopyrimidines and derivatives thereof, thienopyrans and derivatives thereof, pyrimidines and analogs thereof, quinazolinones and analogs thereof, diketenes and analogs thereof, imidazoquinolines and analogs thereof, imidazopyridines and derivatives thereof, thiazolidinediones and derivatives thereof, pyridofuropyrimidines and analogs thereof.

Preferably, the PI3K inhibitor is selected from one or more of IPI-145 (INK1197, duvelisib), a derivative thereof, a pharmaceutically acceptable salt thereof, a solvate thereof, and a prodrug thereof; IPI-145 (INK1197, duvelisib) is a selective PI3Kδ/γinhibitor, and duvelisib has a chemical structure shown in a formula I below:

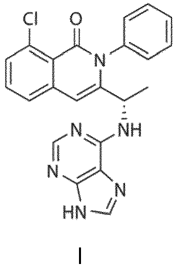

Further, the AKT inhibitors include, but are not limited to, PH domain inhibitors, allosteric inhibitors, and ATP competitive inhibitors, such as MK-2206, GSK690693, Ipatasertib (GDC-0068), and the like.

In one embodiment of the invention, the PI3K/AKT signaling pathway inhibitor is a PI3K/AKT upstream protein inhibitor, preferably selected from one or more of FAK inhibitors, Syk inhibitors and Src inhibitors; the FAK inhibitor is preferably selected from one or more of PF-573228 (a formula II), a derivative thereof, a pharmaceutically acceptable salt thereof, a solvate thereof, and a prodrug thereof, the Syk inhibitor is preferably selected from one or more of R788 (Fostamatinib, a formula III), a derivative thereof, a pharmaceutically acceptable salt thereof, a solvate thereof, and a prodrug thereof; the Src inhibitor is preferably selected from one or more of saracatinib (AZD0530, a formula IV), a derivative thereof, a pharmaceutically acceptable salt thereof, a solvate thereof, and a prodrug thereof, with structural formulas II-IV shown below:

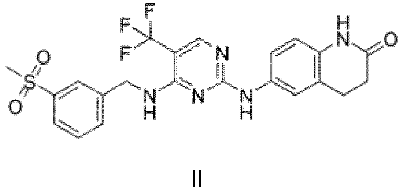

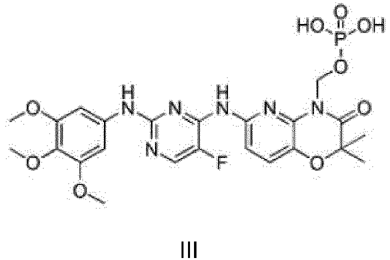

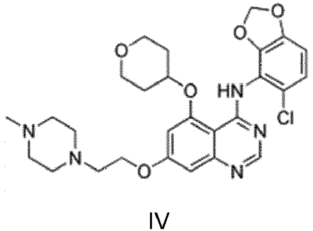

Further, the PI3K/AKT signaling pathway inhibitor is a PI3K/AKT/SOX2 axis inhibitor that modulates sternness of drug-resistant cells; preferably, the PI3K/AKT/SOX2 axis inhibitor is a SOX2 downstream protein inhibitor, wherein a downstream protein is preferably CDK6 and/or FGFR1/2.

In a preferred embodiment of the invention, the PI3K/AKT/SOX2 axis inhibitor is a CDK6 inhibitor that can be selected from, but is not limited to, abemaciclib, Ribociclib, and Palbociclib, most preferably one or more of abemaciclib (LY2835219), a derivative thereof, a pharmaceutically acceptable salt thereof, a solvate thereof, and a prodrug thereof, abemaciclib having a chemical structure shown below in a formula V:

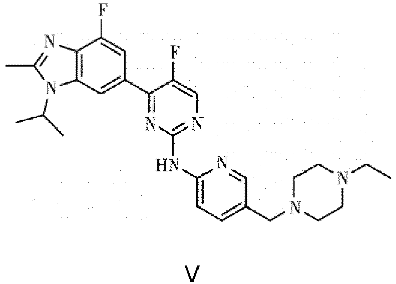

In another preferred embodiment of the invention, the PI3K/AKT/SOX2 axis inhibitor is an FGFR1/2 inhibitor that can be selected from, but is not limited to, BGJ398, AZD4547, PRN1371, LY2874455, and FIIN-2, most preferably one or more of AZD4547, a derivative thereof, a pharmaceutically acceptable salt thereof, a solvate thereof, and a prodrug thereof, AZD4547 having a chemical structure shown below in a formula VI:

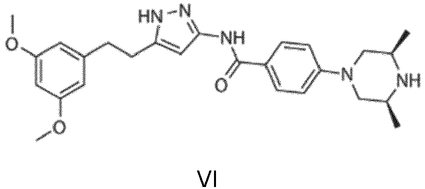

The present invention found that the proportion of CSCs in drug-resistant DLBCL cells was significantly increased, and its sternness was regulated by the activated PI3K/AKT/SOX2 axis. In addition, the PI3K/AKT signaling pathway inhibitor converts CSCs into differentiated tumor cells by reducing SOX2 levels, thereby completely preventing the growth of seeded drug-resistant cells when combined with the R-CHOP regimen.

Based on the results of the above studies, the present invention also provides a pharmaceutical composition for treating B-cell lymphoma, including a PI3K/AKT signaling pathway inhibitor, a monoclonal antibody targeting CD20 and a chemotherapeutic drug in unit preparations of different specifications and a pharmaceutically acceptable carrier or excipient for simultaneous, separate or sequential administration.

Preferably, the monoclonal antibody targeting CD20 is rituximab.

Further, the chemotherapeutic drug may be any one or more chemotherapeutic drugs effective against the tumor. Preferably, the chemotherapeutic drug is selected from one or more of cyclophosphamide, doxorubicin and vincristine; in a preferred embodiment of the present invention, the chemotherapeutic drug is a combination of the aforementioned three drugs and a hormonal drug prednisone.

Further, the present invention provides a pharmaceutical composition for treating B-cell lymphoma, including a first preparation formed by a PI3K/AKT signaling pathway inhibitor and a pharmaceutically acceptable carrier, a second preparation formed by a monoclonal antibody targeting CD20 and a pharmaceutically acceptable carrier, and a third preparation formed by a chemotherapeutic drug and a pharmaceutically acceptable carrier.

Preferably, the monoclonal antibody targeting CD20 is rituximab.

Optionally, the dosage forms of the above preparations are injection administration preparations, gastrointestinal administration preparations, respiratory administration preparations, transdermal administration preparations, mucosal administration preparations, or cavity administration preparations.

Wherein, the injection administration preparations described in the present invention include, but are not limited to, intravenous injection, intramuscular injection, subcutaneous injection, intradermal injection, intracavitary injection, and the like; the gastrointestinal administration preparations described in the present invention refer to pharmaceutical dosage forms which enter the gastrointestinal tract after oral administration and act locally or play a systemic action by absorption and include, but are not limited to, powders, tablets, granules, capsules, solutions, emulsions, suspensions and the like; the respiratory administration preparations described in the present invention include, but are not limited to, sprays, aerosols, powder aerosols, and the like; the transdermal administration preparations described in the present invention include, but are not limited to, topical solutions, lotions, liniments, ointments, plasters, pastes, patches and the like; dosage forms for mucosal administration described in the present invention include, but are not limited to, eye drops, nasal drops, eye ointments, gargles, sublingual tablets, adhesive patches, patches and the like; and the cavity administration preparations described in the present invention include, but are not limited to, suppositories, aerosols, effervescent tablets, drops, dripping pills, and the like.

Preferably, the PI3K/AKT signaling pathway inhibitor is selected from one or more of duvelisib, a derivative thereof, a pharmaceutically acceptable salt thereof, a solvate thereof, and a prodrug thereof.

The present invention also provides a pharmaceutical composition for treating B-cell lymphoma, including a CDK6 inhibitor and a chemotherapeutic drug; and further including a monoclonal antibody targeting CD20, such as rituximab.

Further, the present invention provides a pharmaceutical composition for treating B-cell lymphoma, including a CDK6 inhibitor, rituximab, and a chemotherapeutic drug in unit preparations of different specifications and a pharmaceutically acceptable carrier or excipient for simultaneous, separate or sequential administration.

Preferably, the CDK6 inhibitor is selected from one or more of abemaciclib, a derivative thereof, a pharmaceutically acceptable salt thereof, a solvate thereof, and a prodrug thereof.

The present invention also provides a pharmaceutical composition for treating B-cell lymphoma, including an FGFR1/2 inhibitor and a chemotherapeutic drug; further including a monoclonal antibody targeting CD20, such as rituximab.

Further, the present invention provides a pharmaceutical composition for treating B-cell lymphoma,including an FGFR1/2 inhibitor, rituximab, and a chemotherapeutic drug in unit preparations of different specifications and a pharmaceutically acceptable carrier or excipient for simultaneous, separate or sequential administration.

Preferably, the FGFR1/2 inhibitor is selected from one or more of AZD4547, a derivative thereof, a pharmaceutically acceptable salt thereof, a solvate thereof, and a prodrug thereof.

Further, the present invention also provides use of a PI3K/AKT signaling pathway inhibitor in the treatment of B-cell lymphoma resistant to a chemotherapeutic drug, and in the preparation of a drug for treating B-cell lymphoma resistant to a chemotherapeutic drug; wherein the PI3K/AKT signaling pathway inhibitor includes PI3K inhibitors, AKT inhibitors, and PI3K/AKT upstream protein FAK inhibitors, Syk inhibitors and Src inhibitors, and PI3K/AKT downstream protein CDK6 inhibitors and FGFR1/2 inhibitors.

Further, the present invention also provides use of a chemotherapeutic drug and a PI3K/AKT signaling pathway inhibitor in the treatment of B-cell lymphoma, and in the preparation of a combined drug for the treatment of B-cell lymphoma; wherein the PI3K/AKT signaling pathway inhibitor includes PI3K inhibitors, AKT inhibitors, and PI3K/AKT upstream protein FAK inhibitors, Syk inhibitors and Src inhibitors, and PI3K/AKT downstream protein CDK6 inhibitors and FGFR1/2 inhibitors.

Further, the present invention also provides use of a chemotherapeutic drug, a monoclonal antibody targeting CD20 and a PI3K/AKT signaling pathway inhibitor in the treatment of B-cell lymphoma, and in the preparation of a combined drug for the treatment of B-cell lymphoma; wherein the PI3K/AKT signaling pathway inhibitor includes PI3K inhibitors, AKT inhibitors, and PI3K/AKT upstream protein FAK inhibitors, Syk inhibitors and Src inhibitors, and PI3K/AKT downstream protein CDK6 inhibitors and FGFR1/2 inhibitors.

Wherein, the B-cell lymphoma is diffuse large B-cell lymphoma, or the B-cell lymphoma is B-cell lymphoma resistant to chemotherapeutic drugs, or the B-cell lymphoma is B-cell lymphoma with an elevated proportion of CSCs, or the B-cell lymphoma is B-cell lymphoma with elevated SOX2 stability.

Given the critical role of CSCs in the process of metastasis and drug resistance, the present invention proposes a new PDT strategy to cope with CSCs, namely to determine a key signaling pathway that maintains sternness of tumor stem cells and then induces CSC differentiation by interfering with this pathway. Differentiated cells are eventually sensitive to conventional therapies, such as chemotherapy. In this situation, the PI3K/AKT signaling pathway inhibitor in combination with the R-CHOP regimen achieves a good therapeutic effect on transplanted model mice, and thus this regimen is worth evaluating drug-resistant DLBCL patients in clinical trials.

The concepts, specific structures and resulting technical effects of the present invention will now be further described in conjunction with the accompanying drawings in order to provide a full understanding of the objects, features and effects of the present invention.

### Brief description of the Drawings

Fig. 1 is a schematic flow diagram of constructing drug-resistant DLBCL cells by increasing drug concentration; wherein L, M, and H represent low, medium, and high doses of unused drug, respectively;
Fig. 2 is a graph of a result of confirming resistance of drug-resistant DLBCL cells to R-mediated CDC by complement-dependent cytotoxicity (CDC) assay, wherein the cells were treated with 3.2 µg/mL of rituximab and 20% NHS for 2 hours, data are expressed as mean ± SD, n = 3, and ****P < 0.0001;
Fig. 3 is a graph of a result of confirming resistance of drug-resistant DLBCL cells to chemotherapeutic drugs by a CCK-8 method, wherein the cells were treated with dose escalation of 4-hydroxycyclophosphamide (4-HC), doxorubicin or vincristine for 48 hours, respectively, data are expressed as mean ± SD, and n = 3;
Fig. 4 is a statistical comparison of the number of stem-like spheres in original (ORI) and drug-resistant (R, CHO, RCHO, meaning resistant to R, CHO, or RCHO, respectively) DLBCL cells and photographs thereof, a scale bar: 100 µm, data expressed as mean ± SD, n = 3, ***P < 0.001 and ****P < 0.0001;
Fig. 5 is a comparison graph of the percentage of ALDH1 positive cells in original and drug-resistant DLBCL cells, the percentage of ALDH1 positive cells in drug-resistant DLBCL cells progressively increasing in the order of R, CHO, and RCHO drug-resistant cells compared to original cells, indicating enhanced sternness of drug-resistant DLBCL cells, data expressed as mean ± SD, n = 3, *P < 0.05, **P < 0.01, and ***P < 0.001;
Fig. 6 is a representative image of a result of an Aldefluor assay performed on original and drug-resistant DLBCL cells;
Fig. 7 is a result of immunoblot analysis of expression of stem cell markers CD34, CD133 in original and drug-resistant DLBCL cells;
Fig. 8 is a result of immunoblot analysis of expression ofsternness-associated transcription factors SOX2, OCT4, NANOG, KLF4 and c-Myc expression in original and drug-resistant DLBCL cells, with only SOX2 expression gradually increasing with a trend towards enhanced drug resistance;
Fig. 9 is a comparative image of SOX2 immunohistochemical staining in tissues of a same patient at an initial visit and after relapse in GCB subtype (6 pairs), a scale bar: 20 µm, P: Patient;
Fig. 10 is a comparative image of SOX2 immunohistochemical staining in tissues of a same patient at an initial visit and after relapse in ABC subtype (6 pairs), a scale bar: 20 µm, P: Patient;
Fig. 11 is a comparison result of quantitative scores of SOX2 immunohistochemical staining in tissues of a same patient at an initial visit and after relapse in GCB and ABC subtypes, data expressed as mean ± SD, n = 3, **P < 0.01 and ***P < 0.001;
Fig. 12 is a result of a CDC assay, wherein original LY8 and NU-DUL-1 cells overexpressing SOX2 were treated with R+NHS (empty vector original cells as a control), and SOX2-silenced RCHO-resistant LY8 and NU-DUL-1 cells were treated with R+NHS (SCR-silenced drug-resistant cells as a control), the results showed that aberrant SOX2 expression reduced the susceptibility of original cells to rituximab-mediated CDC, while SOX2 silencing showed an opposite effect in the RCHO-resistant cells, data expressed as mean ± SD, n = 3, **P < 0.01 and ****P < 0.0001;
Fig. 13 is a result of CytoTox-Glo cytotoxicity assay, wherein original LY8 and NU-DUL-1 cells overexpressing SOX2 were treated with CHO (empty vector original cells as a control), and SOX2-silenced RCHO-resistant LY8 and NU-DUL-1 cells were treated with CHO (irrelevant shRNA, i.e. SCR-silenced drug-resistant cells as a control), the results showed that aberrant SOX2 expression reduced the cytotoxic effect of CHO in original cells, while SOX2 silencing restored sensitivity to CHO in RCHO-resistant cells, data expressed as mean ± SD, n = 3, ***P < 0.001 and ****P < 0.0001;
Fig. 14 is a result of a qRT-PCR assay for mRNA levels of SOX2 in original and drug-resistant DLBCL cells, showing that mRNA levels of SOX2 are significantly reduced in nearly all drug-resistant DLBCL cells, data expressed as mean ± SD, n = 3, **P < 0.01, ***P < 0.001, and ****P < 0.0001;
Fig. 15 is a result graph of a signal network generated by RNA-seq based differential gene data using Cytoscape software and its interaction scores in the KEGG database, indicating that a PI3K/AKT signaling pathway and steroid biosynthesis show the highest interaction scores;
Fig. 16 is a graph of GSEA enrichment characteristics of the PI3K/AKT pathway obtained by gene set enrichment analysis performed on RNA-seq-based differential gene data in RCHO-resistant LY8 and NU-DUL-1 cells, with FDR<0.25 considered significant difference;
Fig. 17 is a result of immunoblot analysis of various protein expression (p110α/β/γ/δ, p85, Akt1) and AKT (S473), SOX2 (T118) phosphorylation and SOX2 (K119) methylation in the PI3K/AKT signaling pathway in original and drug-resistant DLBCL cells, showing that PI3K/AKT signaling is activated, thereby promoting SOX2 phosphorylation and reducing SOX2 methylation;
Fig. 18 is a comparative image of p-AKT (S473) immunohistochemical staining in tissues of a same patient at an initial visit and after relapse in GCB subtype (6 pairs), a scale bar: 20 µm, P: patient;
Fig. 19 is a comparative image of p-AKT (S473) immunohistochemical staining in tissues of a same patient at an initial visit and after relapse in ABC subtype (6 pairs), a scale bar: 20 µm, P: patient;
Fig. 20 is a comparison result of quantitative scores of p-AKT (S473) immunohistochemical staining in tissues of a same patient at an initial visit and after relapse in GCB and ABC subtypes, data expressed as mean ± SD, n = 3, **P < 0.01;
Fig. 21 is immunoblot analysis of SOX2 expression of original and drug-resistant DLBCL cells after 8 hours of treatment with 10 µM of MG-132 (ubiquitin proteasome inhibitors), indicating that ubiquitination of SOX2 is reduced in drug-resistant DLBCL cells;
Fig. 22 is a Venn diagram illustrating the number of upregulated genes and the number of overlapping genes thereof in the PK3K/AKT pathway in RCHO-resistant LY8 and NU-DUL-1 cells;
Fig. 23 is a list of top 10 signaling pathways enriched by all 124 up-regulated genes;
Fig. 24 is a result of immunoblot analysis of FAK expression and its phosphorylation in original and drug-resistant DLBCL cells, showing that FAK is activated in drug-resistant LY8 but not activated in drug-resistant NU-DUL-1 cells;
Fig. 25 is a detection result of mRNA levels of integrin subunitsin original and drug-resistant LY8 cells obtained by qRT-PCR, showing that mRNA levels of integrin subunits ITGA1 and ITGB5 are significantly up-regulated in drug-resistant LY8 cells;
Fig. 26 is a result of immunoblot analysis of expression of integrin subunits α1 and β5 in original and drug-resistant LY8 cells, indicating that the integrin subunits α1 and β5 are up-regulated in drug-resistant LY8 cells;
Fig. 27 is a result of immunoblot analysis of the FAK-AKT signaling axis after knockdown of ITGA1 or ITGB5 in LY8-RCHO cells, indicating that knockdown of ITGA1 or ITGB5 inhibits the FAK-AKT signaling axis, thereby leading to degradation of SOX2;
Fig. 28 is a result of immunoblot analysis of the BCR signaling pathway in original and drug-resistant DLBCL cells, indicating that Syk is activated in drug-resistant DLBCL cells by a BCR/Lyn and TCF-3/THP-1 signaling pathway;
Fig. 29 is a result of immunoblot analysis of the BCR-Lyn-Syk-AKT signaling axis after knockdown of CD79A in LY8-RCHO cells, indicating that silencing of CD79A, a constituent subunit of the BCR, inhibits the BCR-Lyn-Syk-AKT signaling axis, thereby leading to degradation of SOX2;
Fig. 30 is a graph of GSEA enrichment characteristics of a chemokine signaling pathway obtained by gene set enrichment analysis performed on RNA-seq-based differential gene data in LY8-RCHO cells, with FDR<0.25 considered significant difference;
Fig. 31 is a list of the top 10 up-regulated genes in the chemokine signaling pathway;
Fig. 32 is a result of detection of mRNA levels of CCR7 in original and drug-resistant LY8 cells obtained by qRT-PCR, showing that mRNA levels of CCR7 are significantly elevated in drug-resistant LY8 cells, data expressed as mean ± SD; N = 3, ***P < 0.001 and ****P <0.0001 ;
Fig. 33 is a result of immunoblot analysis of expression of CCR7 and Src, and Src phosphorylation levels in original and drug-resistant DLBCL cells, indicating that CCR7 expression is up-regulated in CHO and RCHO drug-resistant cells, thus enhancing downstream Src (Y418) phosphorylation;
Fig. 34 is a result of immunoblot analysis of the CCR7-Src-AKT signaling axis after knockdown of CCR7 in LY8-RCHO and NU-DUL-1-RCHO cells, indicating that knockdown of CCR7 inhibits the CCR7-Src-AKT signaling axis, thereby leading to degradation of SOX2;
Fig. 35 is a graph of a comparison result of determining the cell mortality of two RCHO-resistant cells treated with a PI3K inhibitor duvelisib alone, and co-treated with duvelisib and CHO with CytoTox-Glo cytotoxicity assay, showing that duvelisib and CHO used in combination significantly reversed resistance of RCHO-resistant cells to CHO, while duvelisib used alone had a negligible effect on direct induction of cell death, data expressed as mean ± SD, n = 3, *P < 0.05, **P < 0.01, ***P < 0.001, and ****P < 0.0001;
Fig. 36 is a graph of a comparison result of determining the cell mortality of LY8-RCHO resistant cells treated with an FAK inhibitor PF-573228 alone, and co-treated with PF-573228 and CHO with CytoTox-Glo cytotoxicity assay, showing that PF-573228 and CHO used in combination significantly reversed resistance of RCHO-resistant cells to CHO, while PF-573228 used alone had a negligible effect on direct induction of cell death, data expressed as mean ± SD, n = 3, *P < 0.05, **P < 0.01, ***P < 0.001, and ****P < 0.0001;
Fig. 37 is a graph of a comparison result of determining the cell mortality of two RCHO-resistant cells treated with an Syk inhibitor R788 alone, and co-treated with R788 and CHO with CytoTox-Glo cytotoxicity assay, indicating that R788 and CHO used in combination significantly reversed the resistance of RCHO-resistant cells to CHO, while R788 used alone had a negligible effect on direct induction of cell death, data expressed as mean ± SD, n = 3, **P < 0.01, ***P < 0.001, and ****P < 0.0001;
Fig. 38 is a graph of a comparison result of determining the cell mortality of two RCHO-resistant cells treated with a Src inhibitor saracatinib alone, and co-treated with saracatinib and CHO with CytoTox-Glo cytotoxicity assay, showing that saracatinib and CHO used in combination significantly reversed resistance of RCHO-resistant cells to CHO, while saracatinib used alone had a negligible effect on direct induction of cell death, data expressed as mean ± SD, n = 3, **P < 0.01, ***P < 0.001, and ****P < 0.0001;
Fig. 39 is a representative image of FACS analysis used to evaluate membrane expression of CD46, CD55, CD59, and CD20 in original and drug-resistant DLBCL cells;
Fig. 40 is a quantitative thermogram of FACS analysis used to evaluate membrane expression of CD46, CD55, CD59, and CD20 in original and drug-resistant DLBCL cells, with each grid representing the average of three biological replicates, the graph indicating that the membrane expression levels of CD46, CD55, and CD59 were not elevated while the membrane expression levels of CD20 were significantly reduced in R and RCHO resistant DLBCL cells;
Fig. 41 is a result of immunoblot analysis of CD20 expression levels in original and drug-resistant DLBCL cells;
Fig. 42 is a result of detection of relative mRNA levels of CD20 in original and drug-resistant DLBCL cells, indicating a significant decrease particularly in R and RCHO drug-resistant cells, data expressed as mean ± SD, n = 3, *P < 0.05, **P < 0.01, and ***P < 0.001;
Fig. 43 is a representative image and its corresponding quantitative thermogram of FACS analysis used to evaluate membrane expression of CD46, CD55, CD59 and CD20 in original LY8 cells with aberrant SOX2 expression as well as in RCHO-resistant LY8 cells with knockdown of SOX2, each grid representing the average of three biological replicates, and the results indicate that aberrant SOX2 expression reduces the membrane level of CD20 while SOX2 deficiency increases the membrane level of CD20;
Fig. 44 is a representative image and its corresponding quantitative thermogram of FACS analysis used to evaluate membrane expression of CD46, CD55, CD59 and CD20 in original NU-DUL-1 cells with aberrant SOX2 expression as well as in RCHO-resistant NU-DUL-1 cells with knockdown of SOX2, each grid representing the average of three biological replicates, and the results indicate that aberrant SOX2 expression reduces the membrane level of CD20 while SOX2 deficiency increases the membrane level of CD20;
Fig. 45 is a representative image and its corresponding quantitative thermogram of FACS analysis used to evaluate the effect of treatment with a PI3K inhibitor duvelisib and an AKT inhibitor MK-2206 on membrane expression of CD46, CD55, CD59 and CD20 in RCHO-resistant DLBCL cells, each grid representing the average of three biological replicates, showing that expression of CD20 is significantly reduced after treatment with duvelisib or MK-2206, but the other three mCRPs did not;
Fig. 46 is a result of the detection of relative mRNA levels of CD20 in RCHO-resistant DLBCL cells afterinhibition of PI3K/AKT by duvelisib or MK-2206, data expressed as mean ± SD, n = 3, ****P < 0.0001;
Fig. 47 is a result of immunoblot analysis of expression levels of AKT, CD20 and SOX2, and AKT phosphorylation levels in RCHO-resistant DLBCL cells after inhibition of PI3K/AKT by duvelisib or MK-2206;
Fig. 48 shows the effect ofinhibition of PI3K/AKT with duvelisib or MK-2206 on the drug resistance of RCHO-resistant DLBCL cells to R-mediated CDC, determined by CDC assay, wherein cells were treated with 1 µM of duvelisib or MK-2206 for 24 hours, data are expressed as mean ± SD, n = 3, *P < 0.05;
Fig. 49 is a Venn diagram illustrating the number of up-regulated genes and the number of overlapping genes with the SOX2 target in the PK3K/AKT pathway in RCHO-resistant LY8 and NU-DUL-1 cells;
Fig. 50 is a result of immunoblot analysis of CDK6 expression levels in original and drug-resistant LY8 cells, indicating that CDK6 is overexpressed in drug-resistant LY8 cells;
Fig. 51 is a comparative image of CDK6 immunohistochemical staining in tissues of a same patient at an initial visit and after relapse in GCB subtype (6 pairs), a scale bar: 20µm, P: patient, indicating higher expression level of CDK6 in relapsed tissues than in tissues at the initial visit;
Fig. 52 is a comparative image of CDK6 immunohistochemical staining in tissues of a same patient at an initial visit and after relapse in the ABC subtype (6 pairs), a scale bar: 20 µm, P: patient;
Fig. 53 is a comparison result of quantitative scores of CDK6 immunohistochemical staining in tissues of a same patient at an initial visit and after relapse in GCB and ABC subtypes, data expressed as mean ± SD, n = 3, *P < 0.05, NS: no significance;
Fig. 54 is a result of immunoblot analysis of FGFR1/2 expression levels in original and drug-resistant NU-DUL-1 cells, indicating that FGFR1 and FGFR2 are overexpressed in drug-resistant NU-DUL-1 cells;
Fig. 55 is a comparative image of FGFR1 immunohistochemical staining in tissues of a same patient at an initial visit and after relapse in ABC subtype (6 pairs), a scale bar: 20 µm, P: patient, indicating higher expression level of FGFR1 in relapsed tissues than in tissues at the initial visit;
Fig. 56 is a comparative image of FGFR1 immunohistochemical staining in tissues of a same patient at an initial visit and after relapse in GCB subtype (6 pairs), a scale bar: 20 µm, P: patient;
Fig. 57 is a comparison result of quantitative scores of FGFR1 immunohistochemical staining in tissues at the same patient initial visit and after relapse in GCB and ABC subtypes, data expressed as mean ± SD, n = 3, * P < 0.05, NS: no significance;
Fig. 58 is a comparative image of FGFR2 immunohistochemical staining in tissues of a same patient at an initial visit and after relapse in the ABC subtype (6 pairs), a scale bar: 20 µm, P: patient, indicating higher expression levels of FGFR2 in relapsed tissues than in tissues at the initial visit;
Fig. 59 is a comparative image of FGFR2 immunohistochemical staining in tissues of a same patient at an initial visit and after relapse in the GCB subtype (6 pairs), a scale bar: 20 µm, P: patient;
Fig. 60 is a comparison result of quantitative scores of FGFR2 immunohistochemical staining in tissues of a same patient at an initial visit and after relapse in GCB and ABC subtypes, data expressed as mean ± SD, n = 3, *P < 0.05, NS: no significance;
Fig. 61 is a result of immunoblot analysis of AKT1 phosphorylation and the expression levels of AKT, SOX2 and CDK6 after treatment of LY8-RCHO cells with duvelisib, indicating that duvelisib reduces the expression of SOX2 and CDK6 by inhibiting AKT phosphorylation;
Fig. 62 is a result of immunoblot analysis of AKT1 phosphorylation and the expression levels of AKT, SOX2, and FGFR1/2 after treatment of NU-DUL-1-RCHO cells with duvelisib, indicating that duvelisib reduces expression of SOX2 and FGFR1/2 by inhibiting AKT phosphorylation;
Fig. 63 is a CytoTox-Glo cytotoxicity assay result, wherein LY8-RCHO cells were treated with abemaciclib alone or co-treated with abemaciclib and CHO, respectively, and the results show that inhibition of CDK6 with abemaciclib restored sensitivity of RCHO-resistant cells to CHO, while abemaciclib used alone had a negligible effect on induction of cell death, data expressed as mean ± SD, n = 3, ** P < 0.01, *** P < 0.001, and **** P < 0.0001;
Fig. 64 is a CytoTox-Glo cytotoxicity assay result, wherein NU-DUL-1-RCHO cells were treated with AZD4547 alone or co-treated with AZD4547 and CHO, respectively, and the results show that inhibition of FGFR1/2 with AZD4547 restored sensitivity of RCHO-resistant cells to CHO, while AZD4547 used alone had a negligible effect on induction of cell death, data expressed as mean ± SD, n = 3, **** P < 0.0001;
Fig. 65 is a result of a CDC assay, wherein LY8-RCHO cells were pre-treated with abemaciclib for 24 hours and then treated with rituximab and 20% NHS for 2 hours, and compared with the control group, the results show that abemaciclib failed to reverse resistance to R-mediated CDC, data expressed as mean ± SD, n = 3;
Fig. 66 is a result of a CDC assay, wherein NU-DUL-1-RCHO cells were pre-treated with AZD4547 for 24 hours followed by treatment with rituximab and 20% NHS for 2 hours, and compared with the control group, results indicate that AZD4547 instead exacerbates resistance to R-mediated CDC, data expressed as mean ± SD, n = 3, * P < 0.05;
Fig. 67 is a result of immunoblot analysis of OCT4, NANOG, KLF4 and c-MYC, CD34 and CD133 expression levels after overexpression of SOX2 in original LY8 and NU-DUL-1 cells, indicating that aberrant SOX2 expression increases levels of CSC-associated molecules in original cells;
Fig. 68 is a result of immunoblot analysis of OCT4, NANOG, KLF4 and c-MYC, CD34 and CD133 expression levels after knockdown of SOX2 in RCHO-resistant LY8 and NU-DUL-1 cells, indicating that SOX2 deficiency reduces the levels of CSC-associated molecules in RCHO-resistant cells;
Fig. 69 is a graph comparing the proportion of ALDH1+ cells after treatment of LY8-RCHO cells with duvelisib and abemaciclib, and NU-DUL-1-RCHO cells with duvelisib and AZD4547, indicating that duvelisib significantly reduced ALDH1+ subpopulations in LY8-RCHO and NU-DUL-1-RCHO cells, abemaciclib had no effect on ALDH1+ subpopulations in LY8-RCHO cells, and AZD4547 significantly reduced ALDH1+ subpopulations in NU-DUL-1-RCHO cells, but to a greatly reduced extent compared with duvelisib, data expressed as mean ± SD, n = 3, ** P < 0.01, *** P < 0.001;
Fig. 70 is a result of immunoblot analysis of AKT, OCT4, NANOG, KLF4 and c-MYC, CD34 and CD133 expression levels and p-AKT phosphorylation levels after treatment of two RCHO-resistant cells with duvelisib, abemaciclib and AZD4547, respectively, indicating that duvelisib significantly reduced levels of CSC-associated molecules in the RCHO-resistant cells, abemaciclib had no effect on its levels but resulted in a reduction in C-MYC, while AZD4547 also reduced their levels but to a greatly reduced extent compared with duvelisib;
Fig. 71 is a result of cell proliferation at 48 hours and 120 hours after treatment LY8-RCHO cells with duvelisib and abemaciclib, and NU-DUL-1-RCHO cells with duvelisib and AZD4547, determined by EdU incorporation assay, data expressed as mean ± SD, n = 3, * P < 0.05, ** P < 0.01, *** P < 0.001, and **** P < 0.0001;
Fig. 72 shows the tumor growth at day 50 and day 90 after inoculation of LY8-RCHO-Luc cells and being administrated according to Table 1, wherein the tumor mass is expressed by the activity intensity of firefly luciferase, and "X" represents mouse death;
Fig. 73 is a quantitative result of total photon flux in Fig. 72 with data expressed as mean ± SEM (n = 7), * P < 0.05, ** P < 0.01, and *** P < 0.001;
Fig. 74 is a Kaplan-Meier survival curve of mice treated with different drugs after inoculation of LY8-RCHO-Luc cells, data expressed as mean ± SEM (n = 7), * P < 0.05, ** P < 0.01 and *** P < 0.001;
Fig. 75 shows the tumor growth on day 50, 70 and 90 after inoculation of NU-DUL-1-RCHO-Luc cells, wherein the tumor mass is expressed by the activity intensity of firefly luciferase and "X" represents mouse death;
Fig. 76 is a quantitative result of total photon flux in Fig. 75 with data expressed as mean ± SEM (n = 7), * P < 0.05 and ** P < 0.01;
Fig. 77 is a Kaplan-Meier survival curve of mice treated with different drugs after inoculation of NU-DUL-1-RCHO-Luc cells, data expressed as mean ± SEM (n = 7), ** P < 0.01, *** P < 0.001 and **** P < 0.0001;
Fig. 78 is an image after immunohistochemical staining of SOX2 in tumor tissues grown in the different drug treatment groups after inoculation with LY8-RCHO-Luc cells, a scale bar: 20 µm, indicating that duvelisib significantly reduced the expression levels of SOX2 compared with a saline control, but R-CHOP and abemaciclib did not;
Fig. 79 is an image after immunohistochemical staining of SOX2 in tumor tissues grown in different drug treatment groups after inoculation with NU-DUL-1-RCHO-Luc cells, a scale bar: 20 µm, indicating that duvelisib and AZD4547 significantly reduced the expression levels of SOX2 compared with a saline control, but R-CHOP did not;
Fig. 80 is a schematic diagram of a mechanism by which pro-differentiation therapy against CSC reverses resistance to R-CHOP in DLBCL.

### Detailed Description of Embodiments

### Embodiment 1 SOX2 regulates drug resistance of stem cell-like drug-resistant DLBCL cells

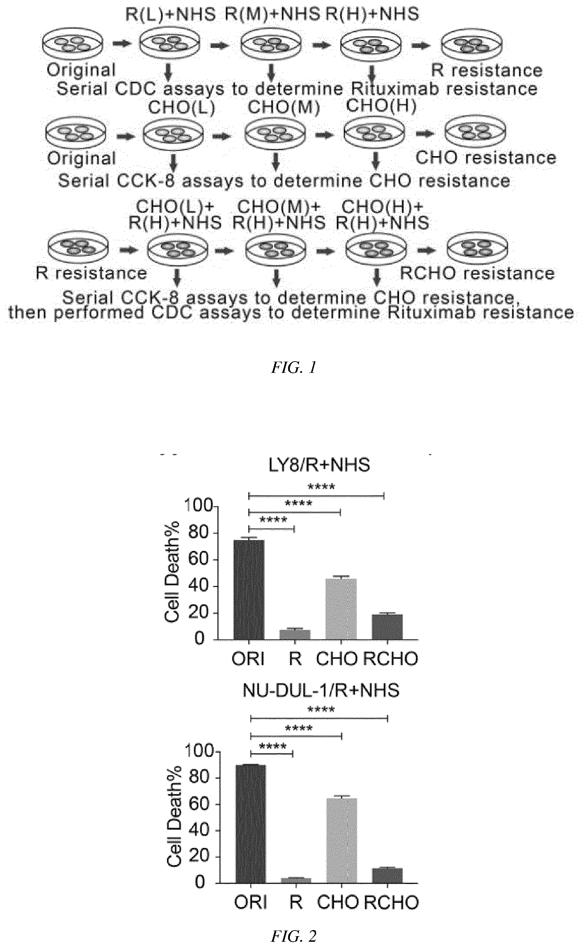

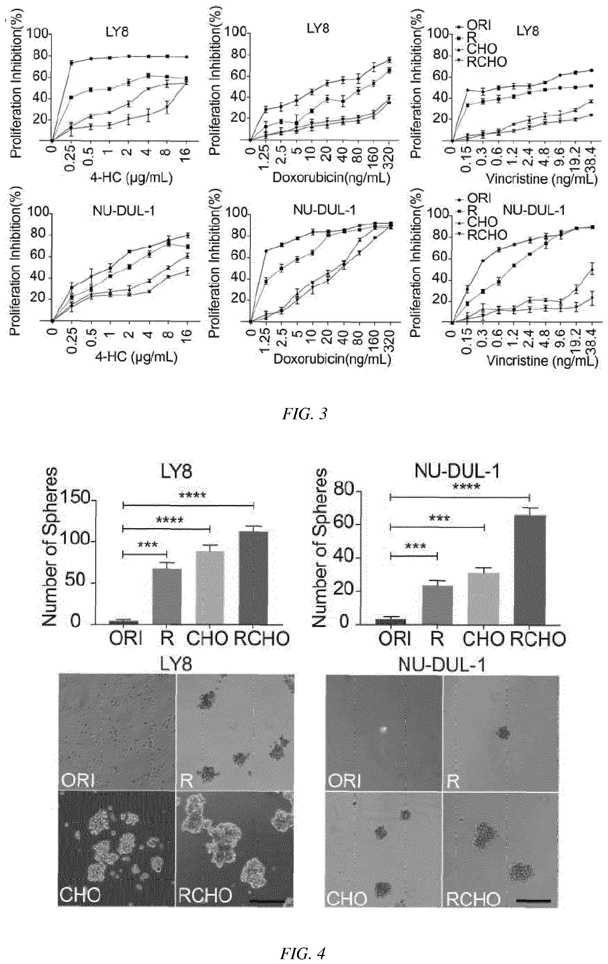

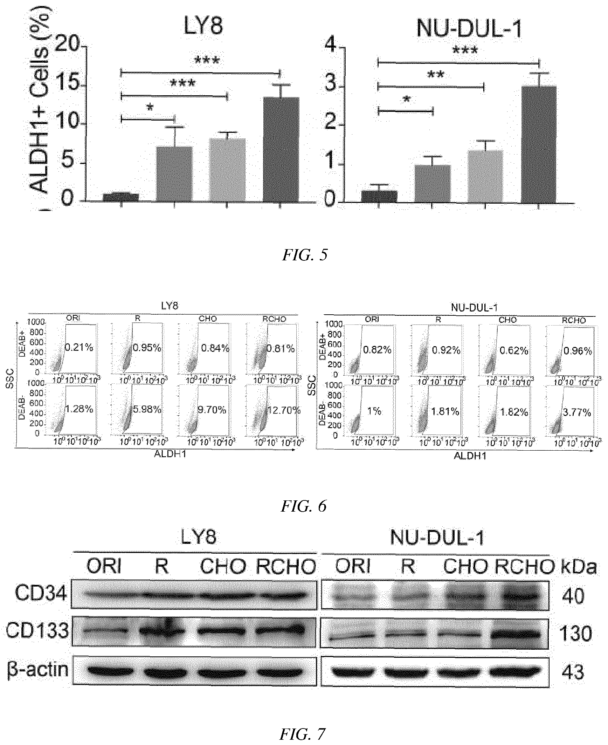

Antibody-dependent cellular cytotoxicity (ADCC) and complement-dependent cytotoxicity (CDC) are major mechanisms of a rituximab therapeutic effect. Tolerance to ADCC may arise from intrinsic characteristics of the individual patient's immune cells; while resistance to CDC is mediated by low expression levels of CD20 and/or high expression levels of membrane-bound complement regulatory proteins (mCRPs), including CD46, CD55 and particularly CD59. CHO chemical drugs inhibit replication of tumor cells by inducing DNA damage or binding to tubulin, and cyclophosphamide must be metabolically activated to 4-hydroxycyclophosphamide (4-HC) in cells to exert this effect. Therefore, by using rituximab plus normal human serum (NHS, as a source of complement), 4-HC plus doxorubicin plus vincristine, and a combination thereof, we constructed DLBCL cells resistant to LY8 (GCB subtype) or NU-DUL-1 (ABC subtype), named R, CHO, and RCHO, respectively (Fig. 1). We validated their resistance to CDC (Fig. 2) and chemotherapeutic drugs (Fig. 3) and, to a certain extent, observed their cross-resistance to rituximab-mediated CDC and to CHO-mediated chemotherapy. Furthermore, compared with original (ORI) cells, drug-resistant cells present stem cell-like spheres of different sizes, and the number of spheres gradually increases in the order of R, CHO, and RCHO (Fig. 4). Although few studies experimentally demonstrate the presence of CSC in NHL, they do exist. Therefore, we used acetaldehyde dehydrogenase 1 (ALDH1) in CSCs of various cancer types and CD34 and CD133 in CSCs of leukemia to determine the sternness of drug-resistant DLBCL cells. The results showed that the proportion of ALDH1+ cells was significantly elevated in drug-resistant cells compared with the original cells (Fig. 5 and Fig. 6), while the expression levels of CD34 and CD133 were increased (Fig. 7).

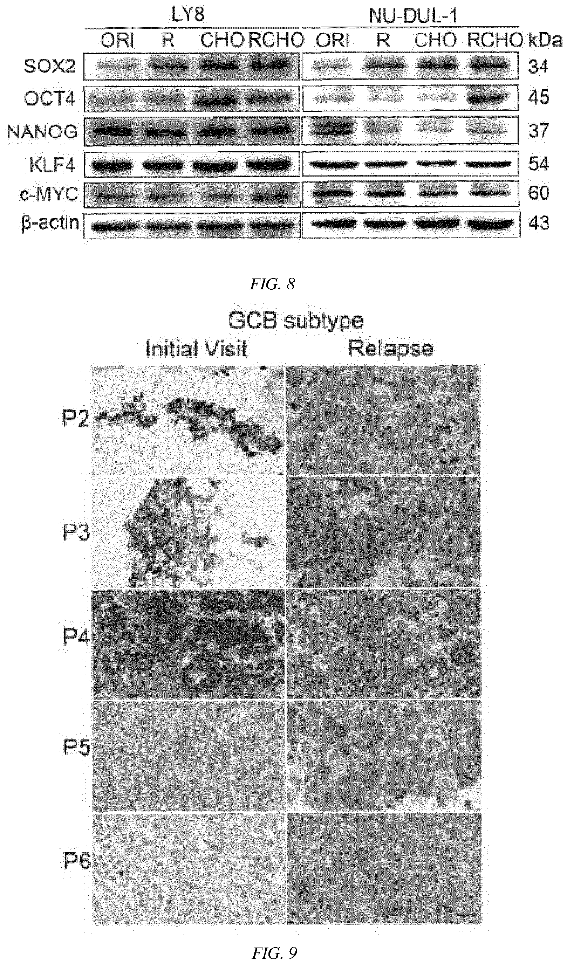

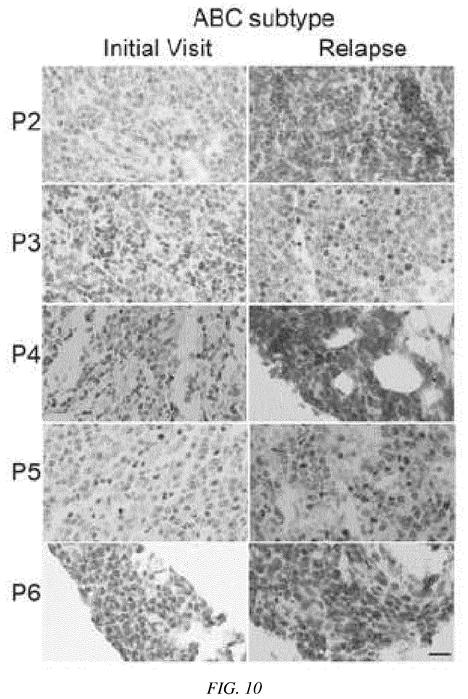

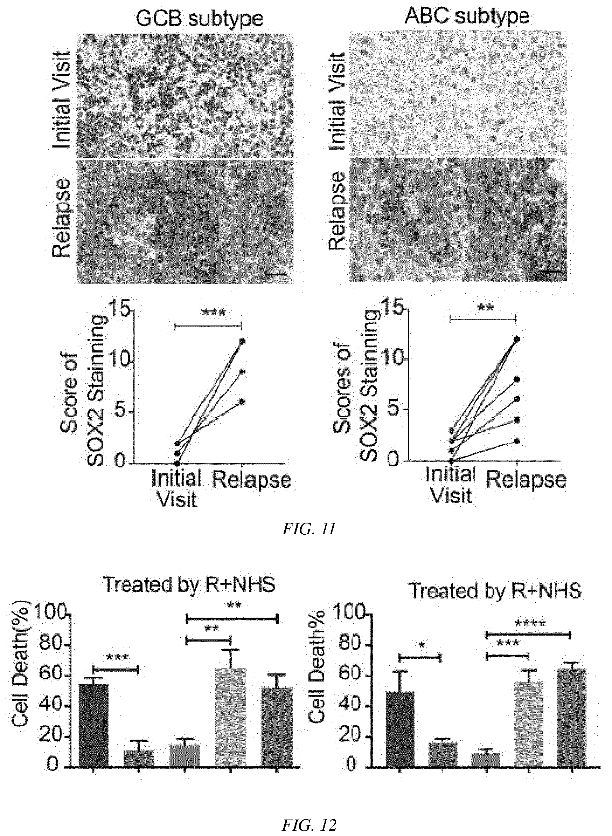

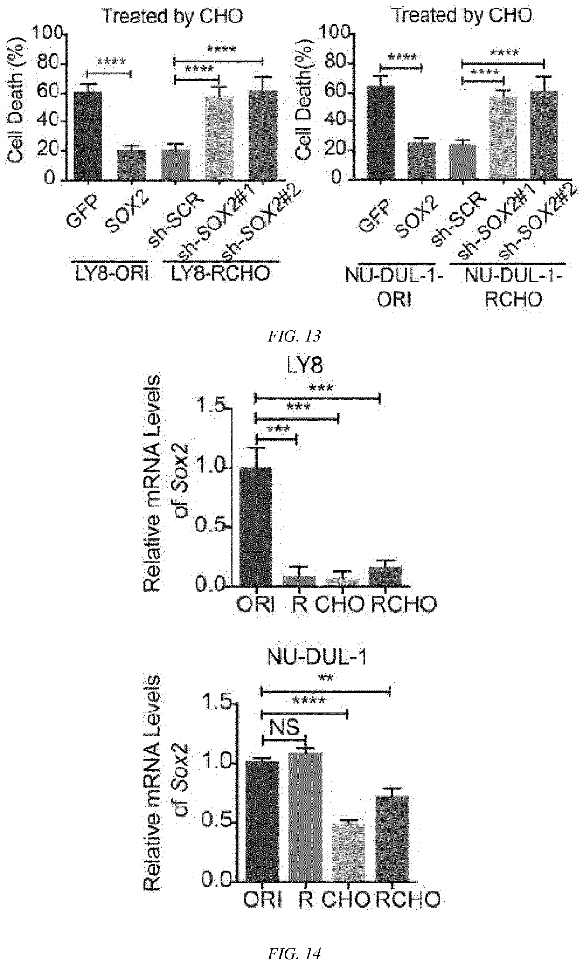

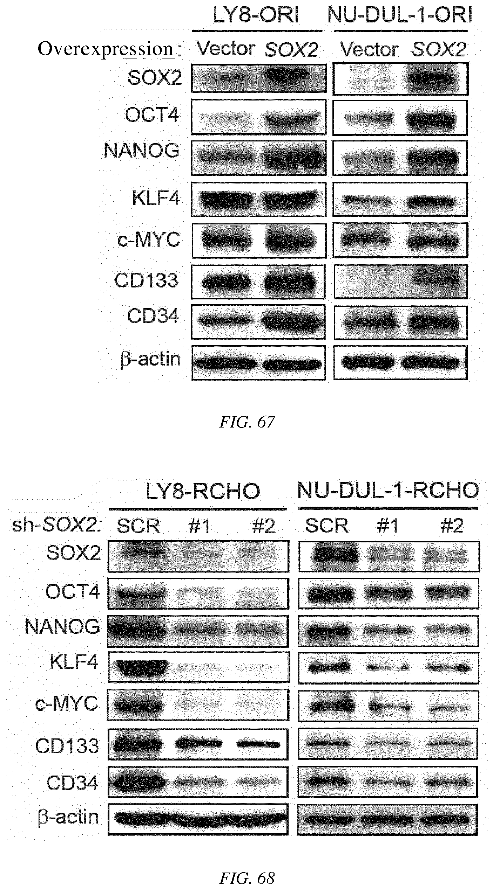

To investigate the molecular regulatory mechanism of sternness in drug-resistant cells, we examined the expression of core sternness-related transcription factors, namely SOX2, OCT4, NANOG, KLF4 and c-Myc. As shown in Fig. 8, only the expression level of SOX2 gradually increased with the increasing trend of drug resistance. Furthermore, in clinical samples from DLBCL patients, we observed that in GCB and ABC subtypes, the immunohistochemical (IHC) staining rate of SOX2 in relapsed tissues was significantly higher than that in their corresponding tissues at the initial visit (Fig. 9, Fig. 10 and Fig. 11). In original LY8 or NU-DUL-1 cells, aberrant SOX2 expression (Fig. 67) significantly reduced cell death induced by rituximab (Fig. 12) or CHO (Fig. 13); in contrast, SOX2 deficiency (Fig. 68) induced opposite effects in RCHO-resistant LY8 or NU-DUL-1 cells (Fig. 12 and Fig. 13). These results indicate that the proportion of CSC is significantly increased in drug-resistant DLBCL cells and the elevation of SOX2 is the cause of drug resistance to rituximab and CHO.

### Embodiment 2 PI3K/AKT signaling phosphorylates and thereby stabilizes SOX2 against ubiquitination-mediated degradation

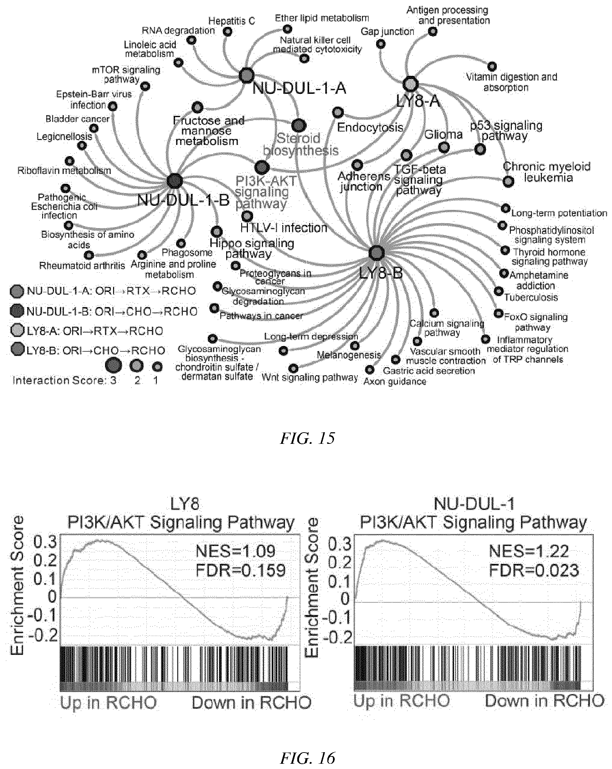

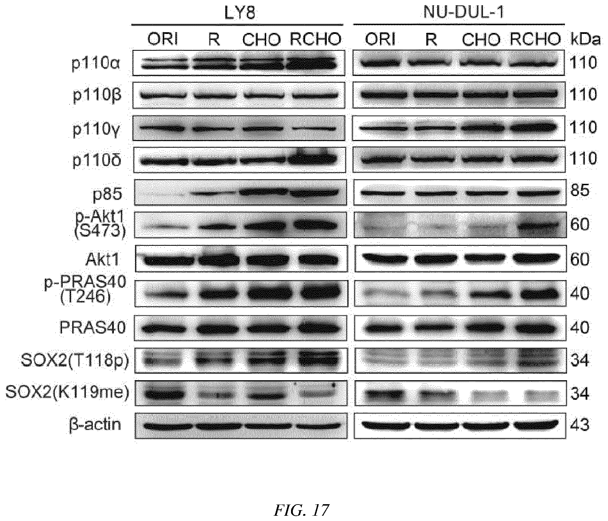

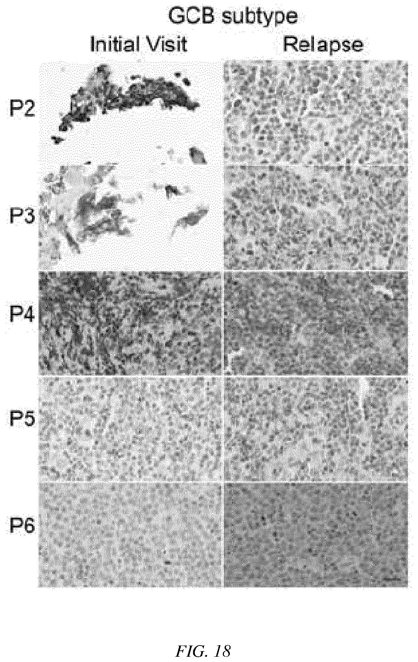

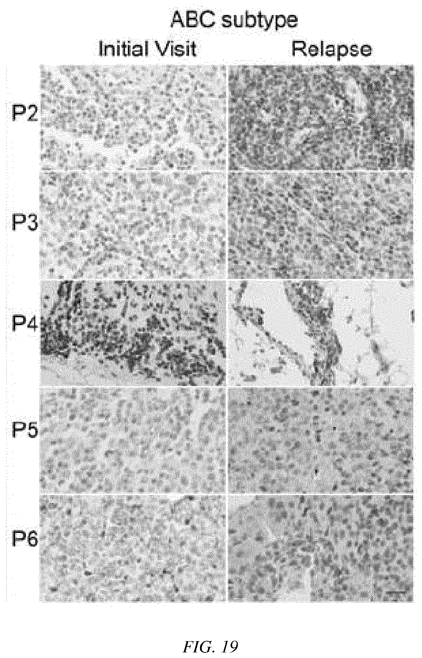

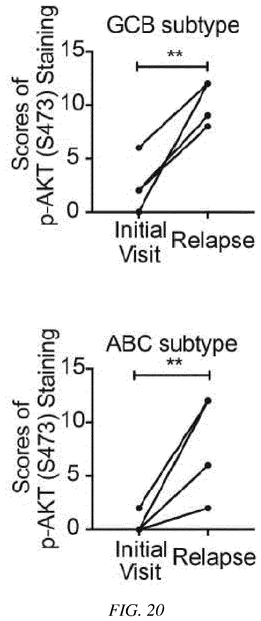

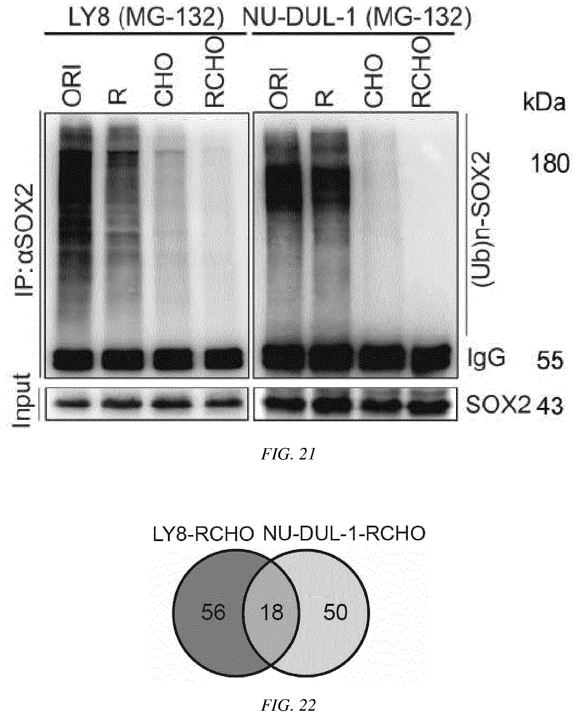

We next investigated the mechanism of SOX2 upregulation in drug-resistant DLBCL cells. Unexpectedly, the mRNA levels of SOX2 decreased significantly in drug-resistant cells, in particular in drug-resistant LY8 cells (Fig. 15), suggesting that transcriptional regulation is not involved in the overexpression of SOX2. Based on RNA-seq data of original cells and drug-resistant cells, interaction scores from KEGG pathway analysis showed that PI3K/AKT signaling pathway and steroid biosynthesis were the most enriched pathways (Fig. 15). Furthermore, gene set enrichment analysis (GSEA) indicated that gene signatures of the PI3K/AKT signaling pathway were significantly activated in the two RCHO-resistant cell lines (Fig. 16). It was experimentally confirmed that phosphorylation of AKT (S473) increased with increase in drug resistance, thereby enhancing phosphorylation of SOX2 (T118) and subsequently suppressing methylation of SOX2 (K119) in the two cell lines; in addition, expression of PI3K subunits p110α/δ and p85 in drug-resistant LY8 cells and expression of p110γ in drug-resistant NU-DUL-1 cells were also elevated (Fig. 17). Correspondingly, for both GCB and ABC subtypes, AKT (S473) phosphorylation was also significantly increased in relapsed biopsies compared with that in tissues of the corresponding patient at the initial visit (Fig. 18, Fig. 19 and Fig. 20). This embodiment demonstrated that the stability of SOX2 was regulated by PI3K/AKT-regulated SOX2 phosphorylation-methylation conversion through the ubiquitination system. Accordingly, ubiquitinated SOX2 was significantly reduced in resistant cells (Fig. 21). Thus, elevated expression of SOX2 in drug-resistant CSC of GCB and ABC subtypes may be caused by AKT-regulated phosphorylation-methylation conversion, further inhibiting ubiquitination and degradation of SOX2.

### Embodiment 3 Multiple signaling events activates the PI3K/AKT pathway in drug-resistant cells

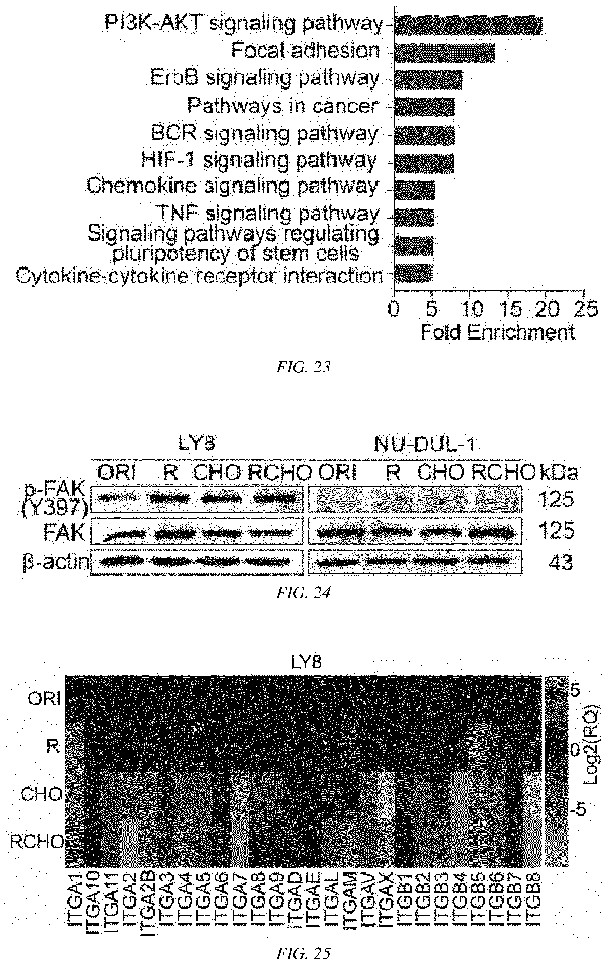

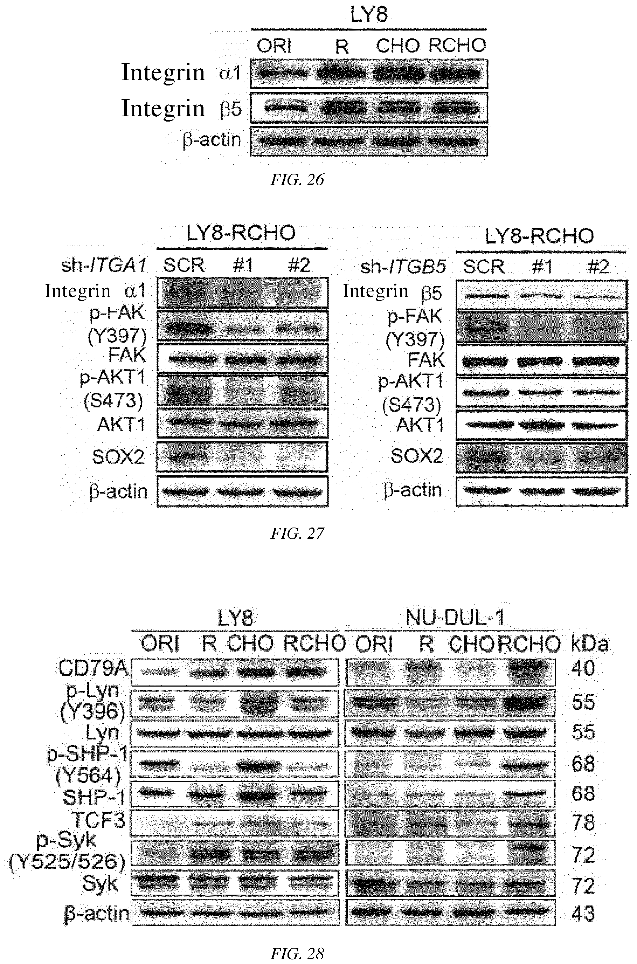

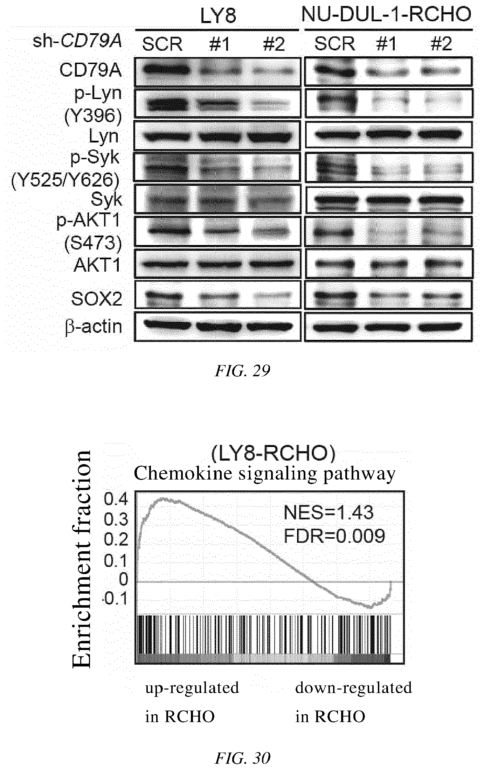

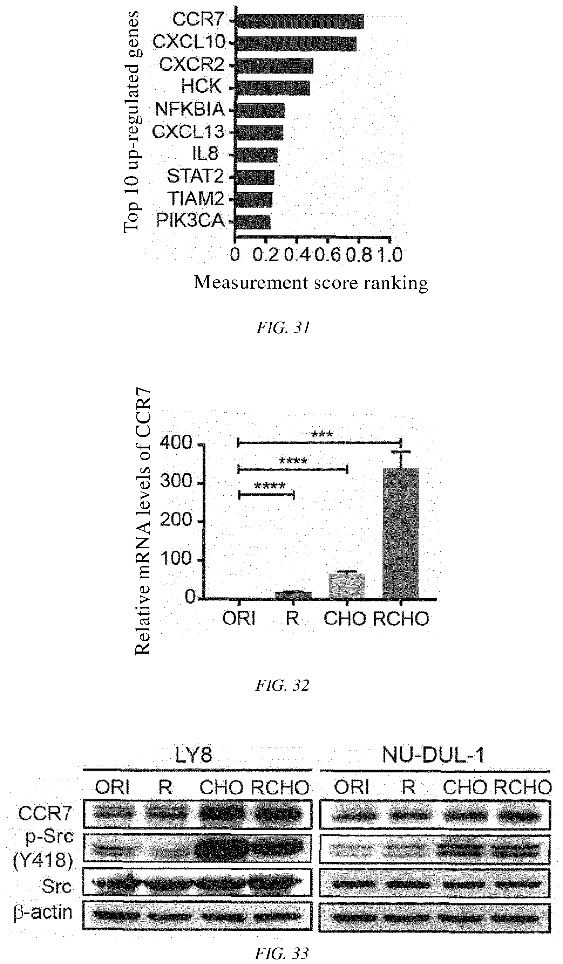

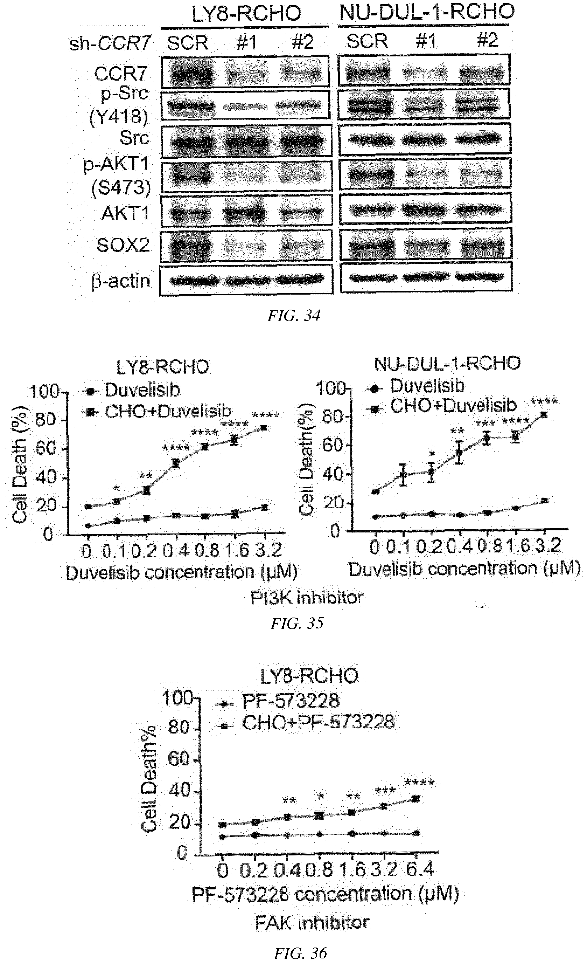

Studies of key regulators upstream of the PI3K/AKT pathway have helped to find potential drug targets that could reduce the expression of SOX2. Therefore, we identified 124 up-regulated genes in the PK3K/AKT pathway (Fig. 16) and the 124 up-regulated genes were classified according to cell subtypes (Fig. 22). Since the number of overlapping genes in both cell subtypes was limited (only 18), we performed KEGG pathway analysis by using all 124 genes. Fig. 23 lists the top 10 pathways, while the PI3K/AKT pathwayis ranked first. PI3Kα/β subtypes are widely expressed, while PI3Kγ/δ subtypes are only expressed in hematopoietic cells, wherein PI3Kδ plays a key role in the development and function of B cells and remains extremely active at all times in B cell malignancies by transducing signals from BCR and other receptors to various integrins and cytokines/chemokines. Consistently, we found that integrin-regulated focal adhesion pathways are ranked second (Fig. 23), however, downstream FAK is only activated in drug-resistant LY8 cells (Fig. 24). Next, we screened the transcriptional levels of all integrin subunits by qRT-PCR and found that only ITGA1 and ITGB5 were significantly elevated in the three drug-resistant LY8 cell lines (Fig. 25), which was confirmed by protein levels (Fig. 26). Our immunoblot analysis on LY8-RCHO cells with knockdown of ITGA1 or ITGB5 showed that deficiency of ITGA1 or ITGB5 significantly inhibited FAK expression and phosphorylation, thereby increasing degradation of SOX2 (Fig. 27). These results indicate that elevated expression of integrin subunits α1 and β5 promotes stabilization of SOX2 by FAK/AKT phosphorylation in drug-resistant LY8 cells. In addition, the BCR signaling pathway was also involved in PI3K/AKT activation (Fig. 23). BCR/Lyn and TCF3/SHP-1 signaling activated Syk in different drug-resistant cells (Fig. 28), but the deficiency of CD79A, a constituent subunit of the BCR signaling pathway, inhibited the BCR/Lyn/Syk/AKT signaling axis, inducing destabilization of SOX2 (Fig. 29). In addition, we further investigated the expression of chemokines, which are thought to activate the PI3K/AKT pathway (Fig. 23). The GSEA results also showed that the chemokine signaling pathway was significantly enriched in RCHO-resistant LY8 cells (Fig. 30), but not in NU-DUL-1 cells; the top 10 related genes are shown in Fig. 31. We confirmed that CCR7 is ranked first and found that mRNA levels of CCR7 were significantly elevated in different drug-resistant LY8 cells, particularly in RCHO-resistant cells (Fig. 32). CCR7 protein levels were also elevated, particularly in CHO and RCHO-resistant LY8 cells (Fig. 33, left); in addition, CCR7 expression was also elevated in drug-resistant NU-DUL-1 cells, but the degree of increase was low (Fig. 33, right). CCR7 deficiency weakened Src/AKT signaling, leading to SOX2 degradation in the two RCHO-resistant cell lines (Fig. 34).

Next, we functionally tested the effect of inhibitors of the above signaling molecules on reversing drug resistance of drug-resistant cells to chemotherapy (CHO). Treatment with the PI3K inhibitor duvelisib alone, if it induced cytotoxicity, was only slightly somewhat cytotoxic; whereas co-treatment with duvelisib and CHO significantly enhanced the cytotoxicity of CHO in the two RCHO-resistant cell lines in a dose-dependent manner (Fig. 35). Similarly, treatment with FAK, Syk, or Src inhibitors alone induced negligible cytotoxicity; however, the combined treatment with FAK, Syk, or Src inhibitors and CHO significantly enhanced the cytotoxicity of CHO, but the degree is much less than that of the combined treatment of CHO with duvelisib (Figs. 36-38). In summary, enhancement of PI3K/AKT signaling in drug-resistant LY8 and NU-DUL-1 cells is induced by a variety of factors, including integrin, BCR, and chemokine signaling, while inhibition of their upstream pathways, other than inhibition of PI3K/AKT, is not effective in reducing survival of drug-resistant cells during chemotherapy.

### Embodiment 4 SOX2-regulated CD20 expression is a decisive factor for rituximab-induced CDC drug resistance

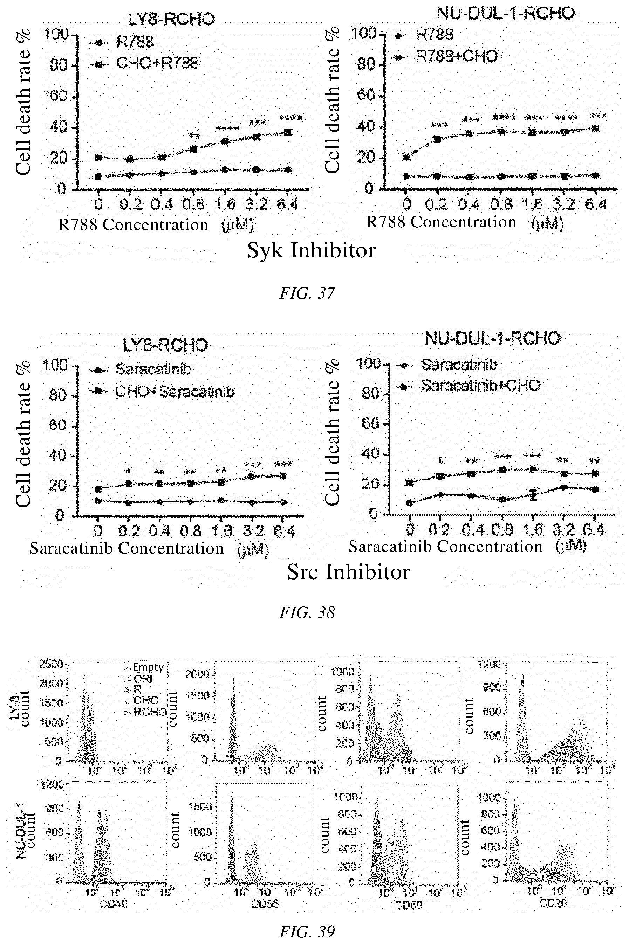

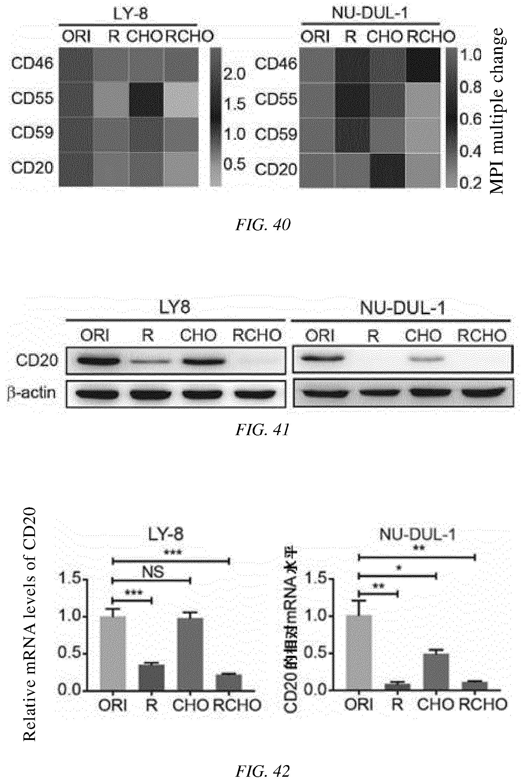

CDC is an important cytotoxic effect of rituximab, and an understanding of the mechanism of the development of drug resistance may be helpful in designing therapeutic drugs to reverse drug resistance. We observed that the membrane expression of CD20 in R and RCHO resistant cells of LY8 and NU-DUL-1 was significantly decreased, while the expression of the other three mCRPs decreased slightly (Fig. 39 and Fig. 40). Results of consistent total CD20 expression levels were obtained with immunoblotting (Fig. 41). Significant reductions in membrane levels of CD20 are likely caused by transcriptional inhibition (Fig. 42).

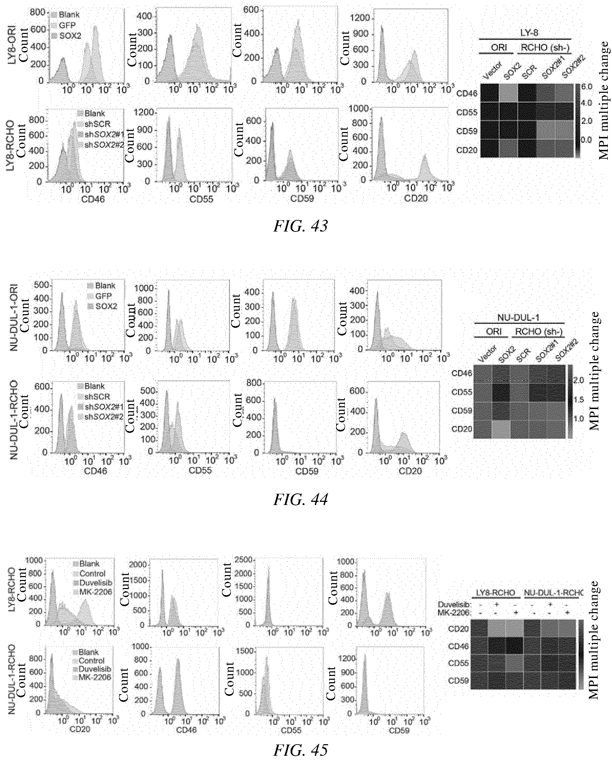

The key role of SOX2 in rituximab-mediated CDC drug-resistant DLBCL has been previously demonstrated (Fig. 12), so we further investigated its underlying mechanism. Abnormal expression of SOX2 significantly reduced CD20 expression in original LY8 and NU-DUL-1 cells, while SOX2 deficiency significantly increased CD20 expression in drug-resistant LY8 and NU-DUL-1 cells (Fig. 43 and Fig. 44). However, the expression of the other three mCRPs did not always correlate with rituximab-mediated CDC drug resistance (Fig. 43 and Fig. 44). These results indicate that expression of CD20 regulated by SOX2, compared with the other mCRPs, is a decisive factor for rituximab-mediated CDC drug resistanceto DLBCL.

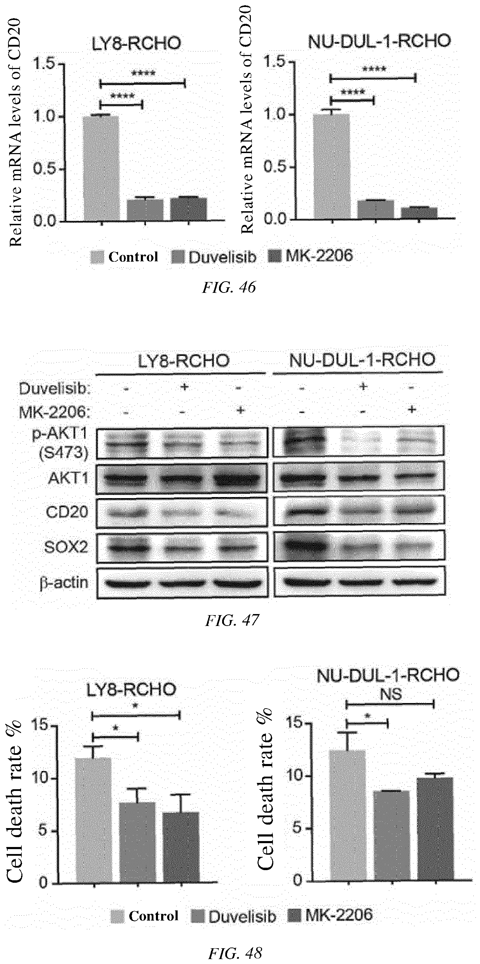

We further determined the effect of the PI3K/AKT signaling pathway inhibitor on rituximab-mediated CDC. In the two RCHO-resistant cell lines, inhibition of PI3K or AKT significantly reduced CD20 expression, while expression levels of the other three mCRPs were not significantly altered (Fig. 45). Similarly, the most likely cause of reduced expression of CD20 was that its transcription was inhibited (Fig. 46). We verified inhibition of AKT and expression of CD20 and SOX2 by immunoblotting (Fig. 47). These results also suggest that regulation of CD20 expression by PI3K/AKT may not be related to SOX2. Subsequently, we found that PI3K/AKT inhibition disrupted rituximab-mediated CDC in RCHO-resistant cells (Fig. 48). Thus, the PI3K/AKT signaling pathway inhibitor may instead exacerbate resistance to rituximab-mediated CDC, as opposed to reversing the effect of resistance to chemotherapy.

### Embodiment 5 GCB and ABC cells employ different SOX2 downstream signaling molecules to generate drug resistance

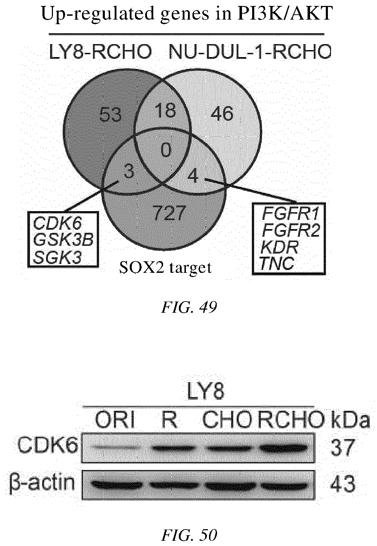

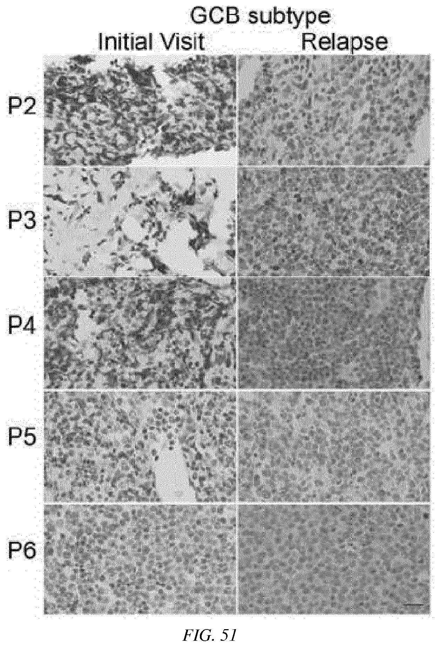

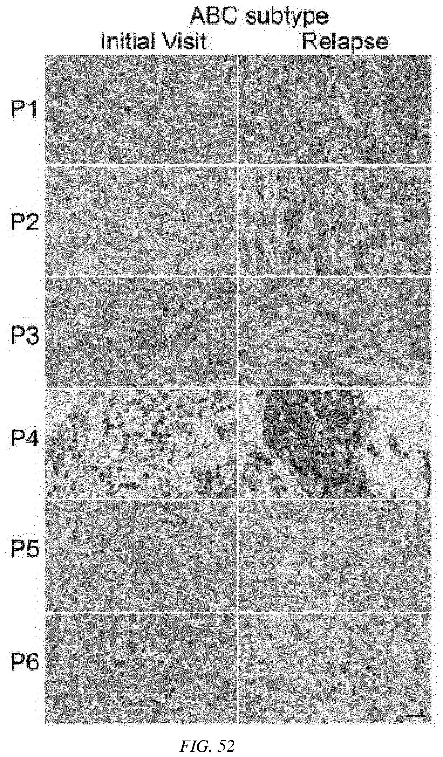

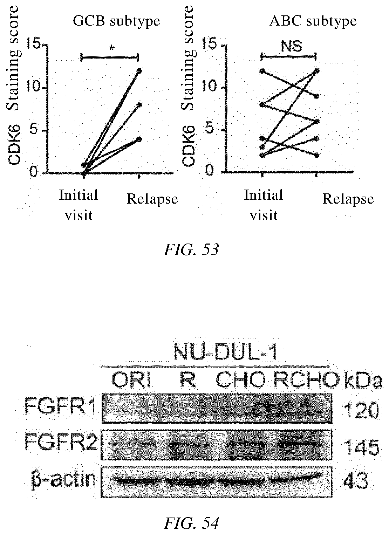

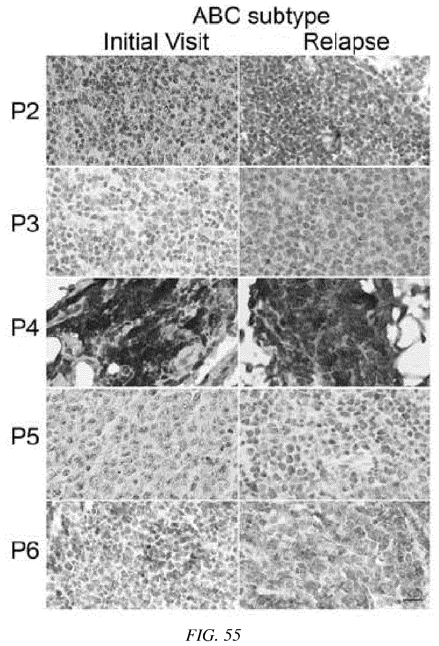

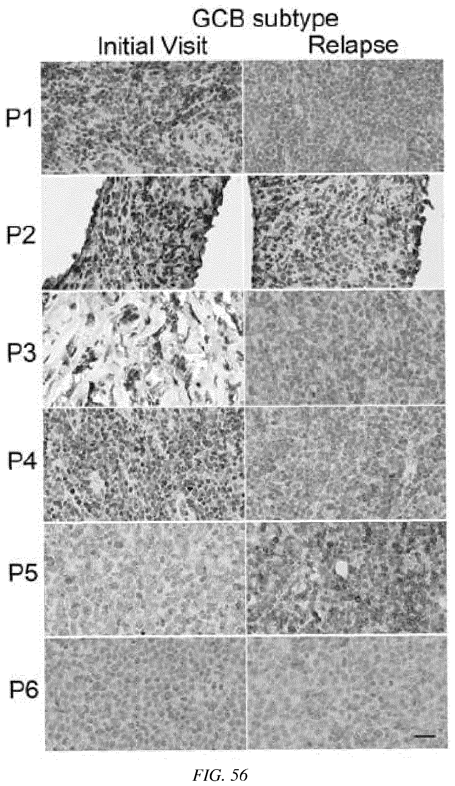

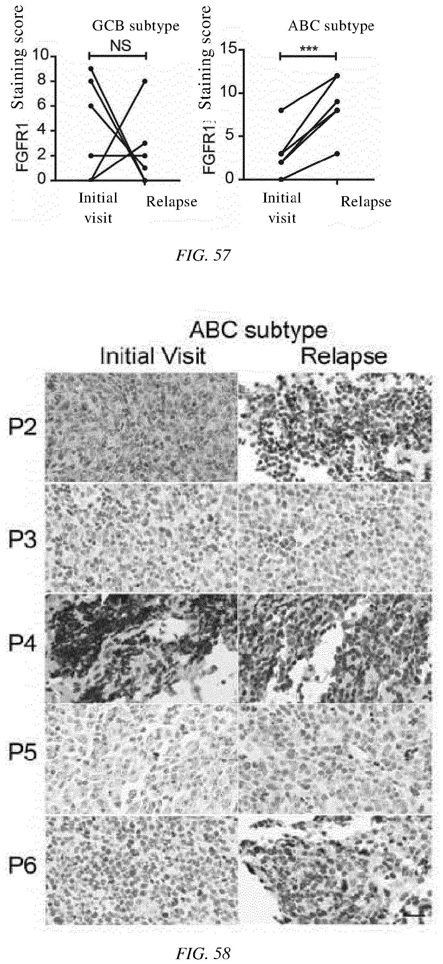

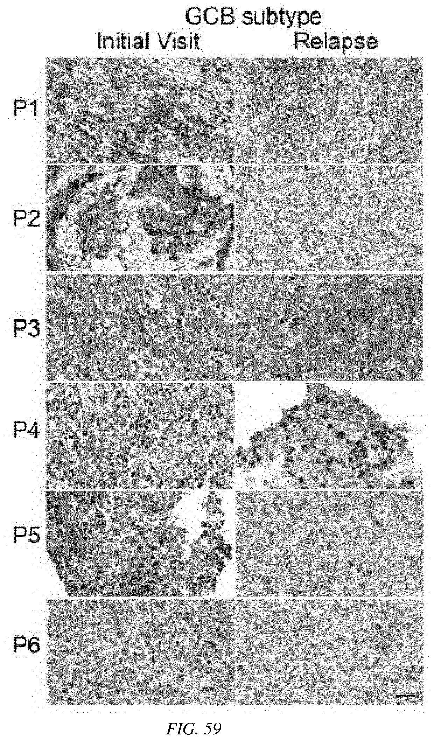

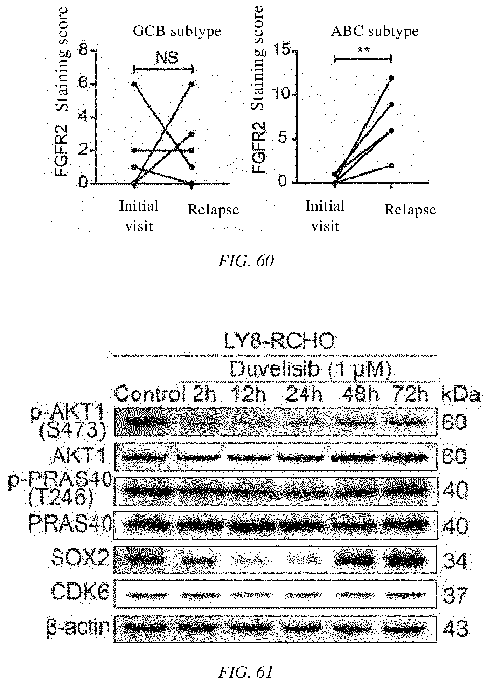

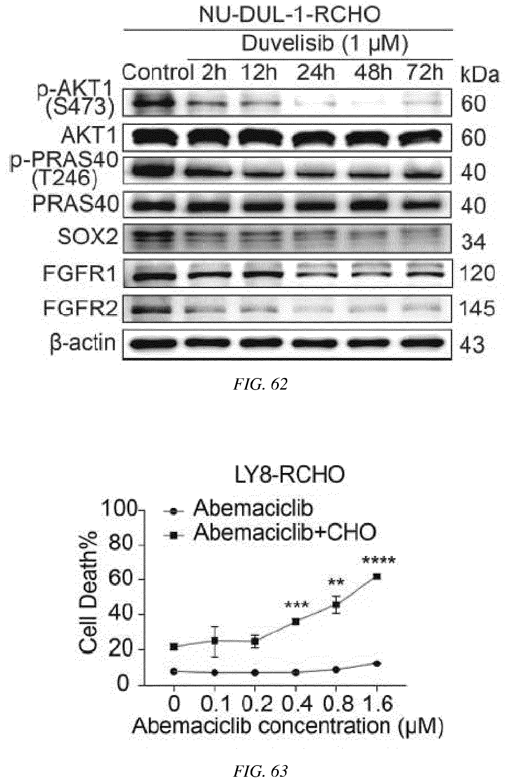

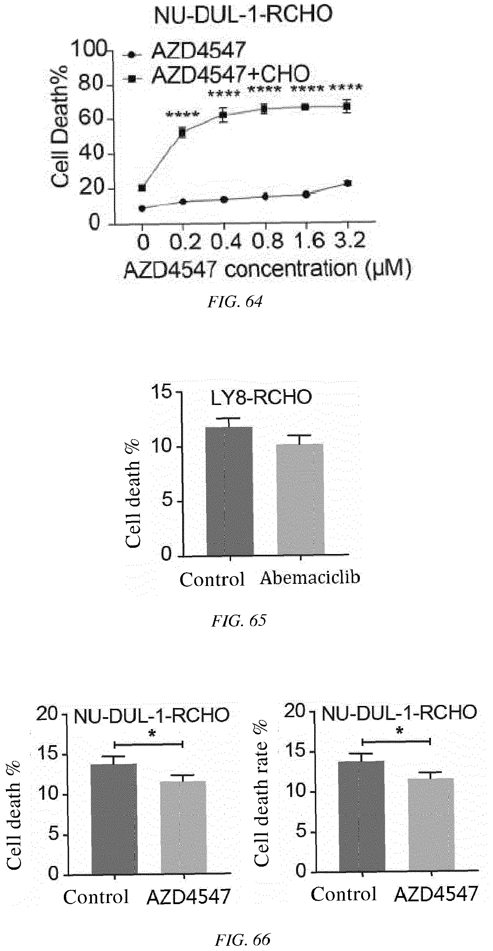

Given the impact of PI3K/AKT inhibition or SOX2 deficiencyon reversing drug resistance to rituximab and/or CHO treatment, we analyzed up-regulated genes in the PI3K/AKT pathway (Fig. 16) and SOX2-targeted genes (molecular signatures database, MSigDB) to identify key targets of SOX2 in RCHO-resistant cells. Three genes CDK6, GSK3B and SGK3 were upregulated in RCHO-resistant LY8 cells, while four genes FCHR1, FGFR2, KDR and TNC were upregulated in RCHO-resistant NU-DUL-1 cells (Fig. 49). Given that CDK4/6 and FGFR inhibitors are undergoing clinical trials in the treatment of a variety of cancers, we subsequently investigated their expression and function. CDK6 is highly expressed in drug-resistant LY8 cells (Fig. 50) and relapsed GCB subtypes, but not in DLBCL tissues of the ABC subtype (Figs. 51-53). Consistently, FGFR1/2 was overexpressed in drug-resistant NU-DUL-1 cells (Fig. 54) and relapsed ABC subtypes, but not in DLBCL tissues of the GCB subtype (Figs. 55-60). Inhibition of PI3K with duvelisib prevented phosphorylation of AKT and induced degradation of SOX2, thereby reducing expression of CDK6 in RCHO-resistant LY8 cells (Fig. 61) or expression of FGFR1/2 in RCHO-resistant NU-DUL-1 cells (Fig. 62). Similar to the effect of PI3K inhibition, inhibition of CDK6 with abemaciclib in RCHO-resistant LY8 cells or inhibition of FGFR1/2 with AZD4547 in RCHO-resistant NU-DUL-1 cells significantly enhanced cytotoxicity of CHO, while the use of these inhibitors alone showed only negligible cytotoxicity on drug-resistant cells (Fig. 63 and Fig. 64). However, CDK6 inhibition in LY8-RCHO resistant cells or FGFR1/2 inhibition in NU-DUL-1-RCHO resistant cells failed to reverse drug resistance to R-mediated CDC (Fig. 65 and Fig. 66). These results indicate that inhibition of SOX2 targets (CDK6 in LY8-RCHO resistant cells and FGFR1/2 in NU-DUL-1-RCHO resistant cells) at least partially reverses drug resistance to chemotherapy but is ineffective for R-mediated CDC.

### Embodiment 6 PI3K/AKT inhibition promotes CSC differentiation by reducing SOX2

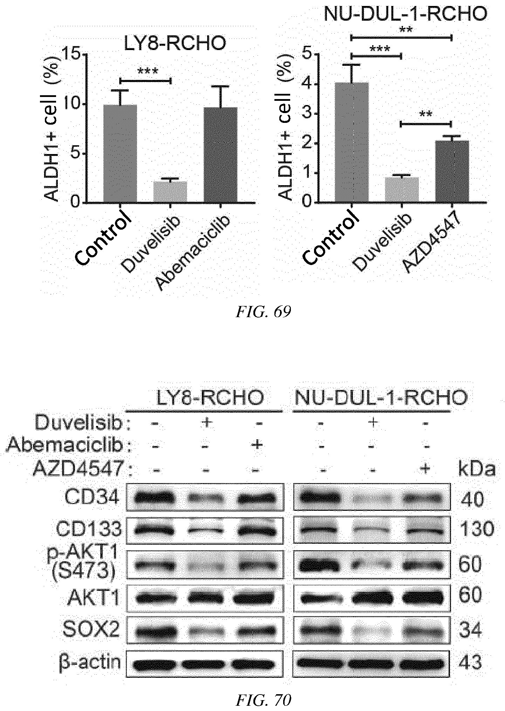

Given the increased proportion of CSC in drug-resistant DLBCL cells and the critical role of SOX2 in RCHO drug resistance, we investigated the role of SOX2 in maintaining its sternness. Aberrant SOX2 expression or SOX2 deficiency significantly increased or inhibited levels of CD34 and CD133 and other sternness-associated transcription factors, including OCT4, NANOG, KLF4 and c-MYC, respectively (Fig. 67 and Fig. 68). These results suggest that SOX2 may be a sternness determining transcription factor in RCHO-resistant DLBCL cells. Furthermore, in the two RCHO resistant cell lines, PI3K inhibition significantly reduced the proportion of CSCs (labeled ALDH1+ cells) (Fig. 69), CDK6 inhibition failed to alter the proportion of CSCs in RCHO resistant LY8 cells (Fig. 69, left), while FGFR1/2 inhibition significantly reduced the proportion of CSCs in RCHO resistant NU-DUL-1 cells, but to a much lesser extent than PI3K inhibition (Fig. 69, right), consistent with previous studies, suggesting that PI3K/AKT may be activated by FGFR signaling. Furthermore, expression levels of CD34, CD133, and sternness-related transcription factors, including SOX2, OCT4, NANOG, KLF4, and c-Myc, were clearly reduced after PI3K inhibition in the two RCHO resistant cell lines, while CDK6 inhibition had no significant effect on expression of the above molecules but resulted in reduction of c-MYC, and expression levels of the above molecules were also reduced after FGFR1/2 inhibition, but to a lesser extent than PI3K inhibition (Fig. 70). Notably, FGFR1/2 upregulates and participates in the activation of PI3K/AKT signaling; therefore, their inhibition reduced the phosphorylation of AKT and reduced the stability of SOX2 (Fig. 70). These results reveal that inhibition of PI3K/AKT directly with duvelisib or indirectly with AZD4547 can promote differentiation of CSCs.

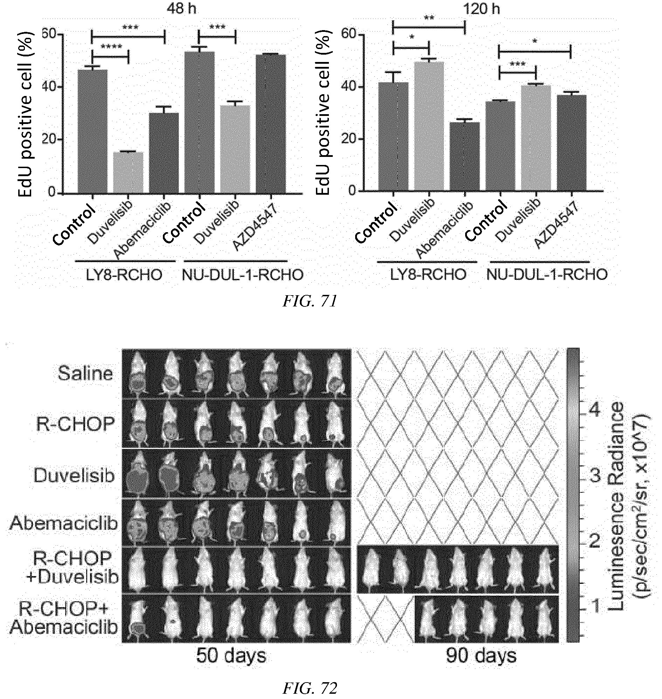

Differentiated tumor cells grow faster than CSCs. Therefore, we functionally examined the effect of PI3K inhibition on cell proliferation by using an EdU incorporation assay. After 48 hours of treatment with duvelisib, direct PI3K inhibition strongly prevented proliferation of LY8-RCHO and NU-DUL-1-RCHO cells and significantly promoted proliferation of cells at 120 hours (Fig. 71). This result revealed a dynamic progression of differentiation from CSC to differentiated cells induced by duvelisib. Inhibition of CDK6 by abemaciclib prevented growth of LY8-RCHO cells at both 48 hours and 120 hours due to the absence of inhibition of PI3K/AKT signaling (Fig. 71). Inhibition of FGFR1/2 by AZD4547 promoted proliferation of NU-DUL-1-RCHO cells due to involvement in inhibition of PI3K/AKT signaling, but to a lesser extent than PI3K inhibition (Fig. 71, right). These results suggest that CDK6 or FGFR1/2 inhibitors and PI3K inhibitors may employ different mechanisms to reverse drug resistance to chemotherapy. PI3K/AKT inhibition efficiently converts CSCs into differentiated cells, whereas CDK6 inhibition may directly prevent the growth of CSCs through cell cycle targeting, whereas FGFR1/2 may exert a dual effect on CSCs. All of these interventions ultimately enhance the sensitivity of CSC to chemotherapy with different efficacy.

### Embodiment 7 Pre-differentiation therapy of CSC formed by combining PI3K inhibitors with R-CHOP completely inhibits tumor growth of RCHO resistant cells

Construction of a transplantation model: we transduced LY8-RCHO cells and NU-DUL-1-RCHO cells with lentiviruses overexpressing the firefly luciferase gene to generate stable cells expressing firefly luciferase, referred to as LY8-RCHO-Luc cells and NU-DUL-1-RCHO-Luc cells, respectively. 8-week-old female SCID mice were purchased from SLAC (Shanghai laboratory animal center). The LY8-RCHO-Luc cells and NU-DUL-1-RCHO-Luc cells were resuspended in PBS prior to intraperitoneal injection of 1.5 x 107 cells per mouse. After intraperitoneal injection of the LY8-RCHO-Luc cells, mice were randomized into 6 groups (7 mice in every group) and given saline, R-CHOP, duvelisib, abemaciclib, R-CHOP + duvelisib, and R-CHOP + abemaciclib, respectively. Mice inoculated with the NU-DUL-1-RCHO-Luc cells were randomly divided into 6 groups (7 mice in every group), and were given saline, R-CHOP, duvelisib, AZD4547, R-CHOP + duvelisib, and R-CHOP + AZD4547, respectively. The drug dosing method based on one course of clinical use is shown in Table 1; the R-CHOP regimen was clinically used to treat DLBCL, while duvelisib, abemaciclib, and AZD4547 were tested in clinical trials (associated NCT numbers are NCT02576275, NCT01739309, and NCT01739309, respectively). Tumor growth was monitored by bioluminescence at day 50 and day 90 after inoculation. For in vivo luminescence imaging, D-luciferin (Promega, Madison, WI) was intraperitoneally injected into mice (150 mg/kg). After 10 minutes, the mice were anesthetized by intraperitoneal injection of pentobarbital (50 mg/kg) and then their bioluminescence intensity was examined by using an In Vivo MS FX PRO system (Bruker Corporation, Billerica, MA). Luminescence images were captured with an exposure time of 30 seconds and the signal intensity of the tumor was measured by using Bruker MI software. The survival time of each mouse was recorded. Surviving mice were euthanized and dissected 120 days after inoculation and no intraperitoneal tumors were found. Tumor tissue was collected from moribund mice and fixed with 4% formalin. All animal experiments were performed in strict accordance with the experimentprotocol approved by the animal ethics committee of Shanghai school of medicine of Fudan university.

**Table 1 Administration modes in the transplantation model**

| **Medicine** | **Dose (mg/kg)** | **Date of Administration (Several days after inoculation)** | **Routes of Administration** |
|---|---|---|---|
| Rituximab (R) | 118.4 | 8 | Intraperitoneal injection |
| Cyclophosph amide (C) | 235.6 | 8 | Intraperitoneal injection |
| Doxorubicin (H) | 16 | 8 | Intraperitoneal injection |
| Vincristine (O) | 0.444 | 8 | Intraperitoneal injection |
| Prednisone (P) | 12.3 | 8, 9, 10, 11, 12 | Intraperitoneal injection |
| Duvelisib | 5.18 | 8, 10, 12, 13, 15, 17, 19, 21, 23, 25, 27 | Gavage |
| Abemaciclib | 41.1 | 8, 10, 12, 13, 15, 17, 19, 21, 23, 25, 27 | Gavage |
| AZD4547 | 32.8 | 8, 9, 10, 11, 12, 13, 14, 15, 16, 17, 18, 19, 20, 21 | Gavage |

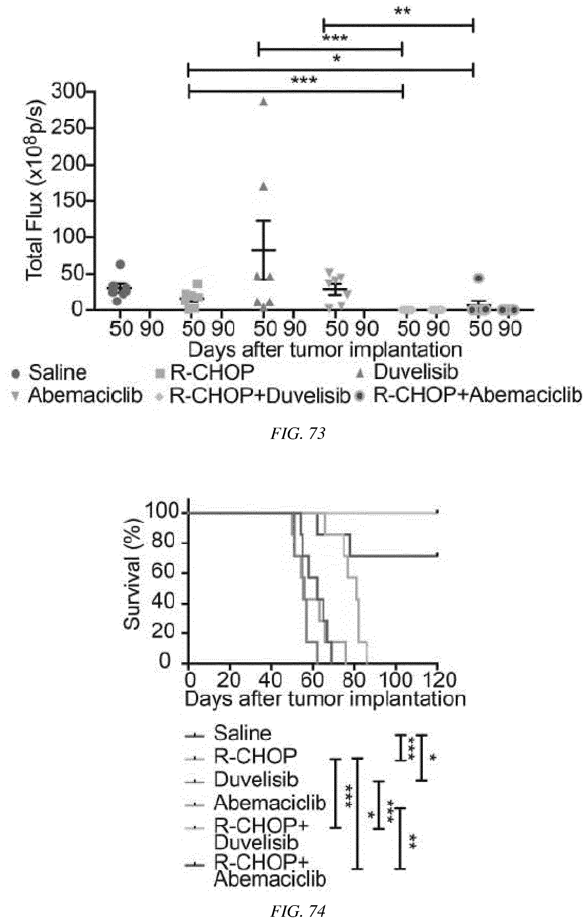

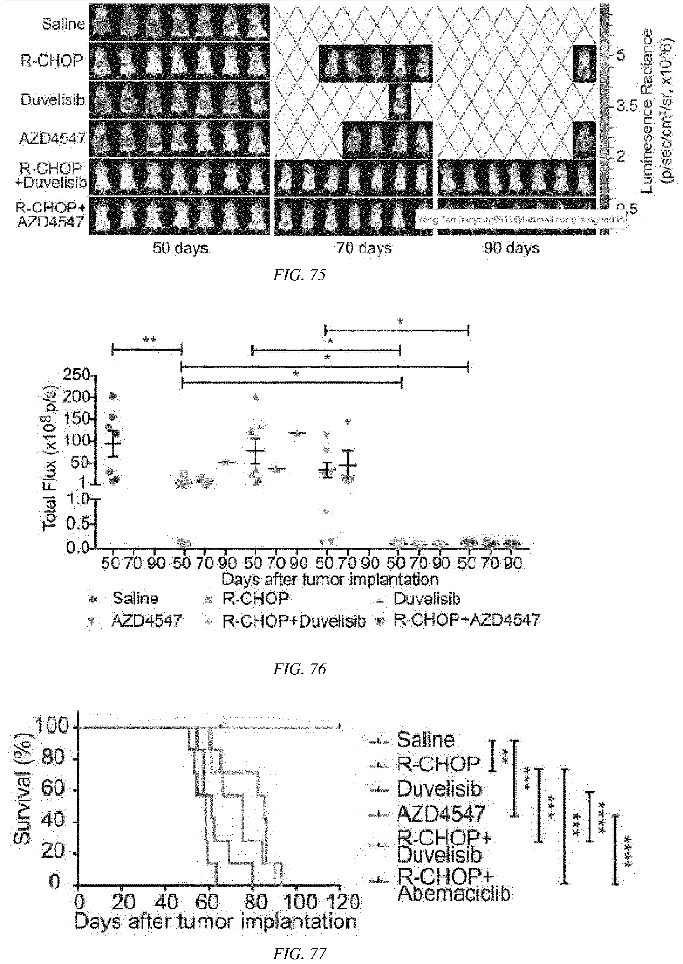

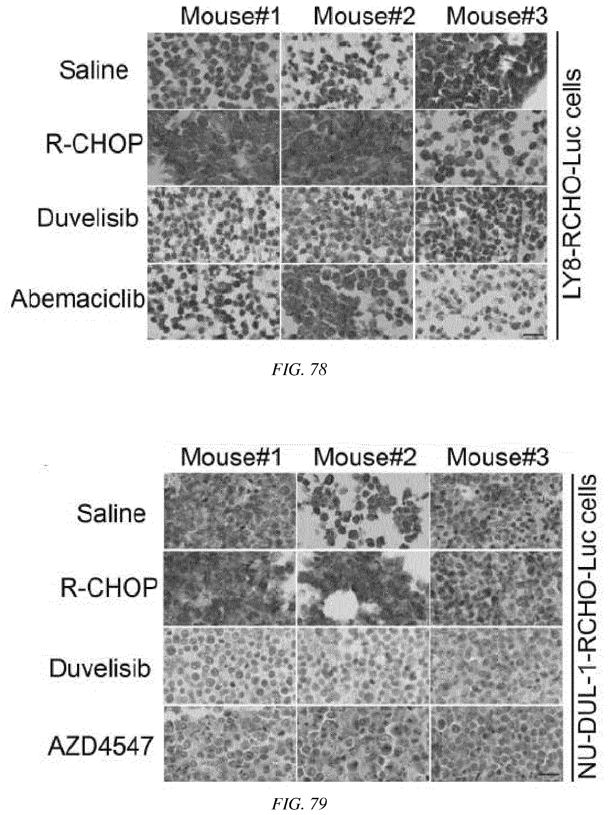

RCHO-resistant LY8 cells and NU-DUL-1 cells transduced with a plasmid expressing luciferase were implanted separately into immunodeficient SCID mice and various treatment regimens were applied. It can be seen from Figs. 72-74 that in mice bearing RCHO-resistant LY8 cells, while R-CHOP significantly prolonged the survival rate (Fig. 74), R-CHOP treatment had no efficacy on tumor growth compared with the saline control (Fig. 72 and Fig.73). These results demonstrate that the in vitro drug resistance of LY8 cells to RCHO is highly reproduced in mice. Unfortunately, neither duvelisib nor abemaciclib alone inhibited tumor growth or prolonged the survival rate (Figs. 72-74); on the contrary, duvelisib accelerated tumor growth compared with normal saline, although this difference was not statistically significant (Fig. 72 and Fig. 73), but resulted in significantly shortened survival rate (Fig. 74). in contrast, when combined with R-CHOP, duvelisib or abemaciclib significantly inhibited tumor growth and prolonged the survival rate compared with R-CHOP or an inhibitor alone (Figs. 72-74). More importantly, combination treatment of R-CHOP with duvelisib completely inhibited tumor development and all treated mice survived until the end of the experiment at day 120, compared with this, two mice receiving combination treatment with abemaciclib died at day 62 and 78 (Figs. 72-74). As can be seen from Figs. 75-77, similar but not identical results were obtained in mice bearing RCHO-resistant NU-DUL-1 cells. R-CHOP significantly inhibited tumor growth and prolonged the survival time (Figs. 75-77), likely due to weaker sternness and decreased proportion of CSCs, resulting in weaker resistance to RCHO than RCHO-resistant LY8 cells (Figs. 4, 5, and 7). Duvelisib used alone also had no efficacy on tumor growth and the survival time, while AZD4547 used alone significantly prolonged the survival time (Figs. 75-77). Importantly, when used in combination with R-CHOP, both duvelisib and AZD4547 could completely inhibit tumor growth and prolong the survival time to the end of the experiment at day 120 (Figs. 75-77). Different tumor-inhibiting effects of duvelisib, abemaciclib, and AZD4547 further support their potential distinct drug resistance reversal mechanisms; i.e. duvelisib converts CSCs into differentiated cells, abemaciclib inhibits CSC growth, and AZD4547 exhibits dual effects on CSCs. SOX2 staining of tumor tissues grown after inoculation treated with different drugs supports this finding. Duvelisib or AZD4547 significantly reduced the expression of SOX2, but R-CHOP or abemaciclib did not (Fig. 78 and Fig. 79). Thus, the success of this combination therapy is likely due to the conversion of CSCs into differentiated cells through PI3K/AKT/SOX2 axis inhibition.

### Embodiment 8 Specific methods for related experiments

### 1. DLBCL tissue sample collection

We investigated the medical history of all DLBCL patients from 2008 to 2015 at Shanghai cancer center of the Fudan university, with a total of 24 patients simultaneously receiving paraffin embedding of tissue specimens at the initial visit and at relapse. DLBCL cases were classified into molecular subtypes of GCB (12 cases) or ABC (12 cases) based on Hans immunohistochemical algorithm. Relapsed patients received at least 6 courses of R-CHOP therapy. The first biopsy is to make a diagnosis at the initial visit, while the second biopsy is used to determine whether it is a relapsed status or a primary diagnosis. The time interval between two biopsies is different for every patient. These DLBCL samples were subsequently collected for IHC staining. The ethics committee of Shanghai cancer center of the Fudan university approved this study and all patients signed informed consents.

### 2. Cell culture and reagents

The human DLBCL cell line GCB subtype OCI-LY8 and ABC subtype NU-DUL-1 were from the department of pathology, Shanghai cancer centerof the Fudan university (Shanghai, China). LY8 cells were cultured in an IMDM (Iscove's modified Dulbecco's medium) medium containing 10% (v/v) of fetal bovine serum and 1% (v/v) of penicillin/streptomycin. NU-DUL-1 cells were cultured in an RPMI 1640 medium containing 10% of fetal bovine serum and 1% of penicillin/streptomycin.

As a source of supplements, normal human serum (NHS) was collected from 10 healthy humans, mixed and sub-packaged and stored at-80°C until use. Heat inactivated human serum (IHS) prepared by incubation in a 65°C water bath for 30 minutes was used as a negative control prior to use.

Prior to the experiment, polyclonal antibodies against SOX2-T118p and SOX2-K119me were prepared. Information for the commercial antibodies used in this study is listed in Table 2.The PI3K inhibitor IPI-145 (duvelisib), the CDK6 inhibitor abemaciclib, the FGFR1/2 inhibitor AZD4547, the FAK inhibitor PF-573228, the Syk inhibitor R788, the Src inhibitor saracatinib, and prednisolone were purchased from MedChem Express Company (Monmouth Junction, NJ).

### 3. Construction and characterization of RCHO-resistant DLBCL cells

We constructed LY8 cells and NU-DUL-1 cells that are resistant to rituximab-mediated complement-dependent cytotoxicity (CDC). Briefly, original LY8 (LY8-ORI) cells and original NU-DUL-1 (NU-DUL-1-ORI) cells were treated with rituximab containing 20% (v/v) of NHS (Roche, Basel, Switzerland) with a concentration of gradually increasing from 4 µg/mL to 32 µg/mL to construct drug-resistant cells. Rituximab-resistant cells are represented by LY8-R and NU-DUL-1-R. LY8-R and NU-DUL-1-R cells were treated with 32 µg/mL of rituximab containing 20% (v/v) of NHS every 21 days to maintain drug resistance. Cytolysis induced by CDC was evaluated by detection of positive cells stained with propidium iodide (PI) through fluorescence activated cell sorting (FACS).

Further, we constructed LY8 and NU-DUL-1 cells resistant to chemotherapeutic drugs. Briefly, LY8-ORI and NU-DUL-1-OR1 cells were treated with doxorubicin (Selleck Chemicals, Houston, TX) and vincristine (Selleck Chemicals, Houston, TX) at a clinical ratio of 50:1.4 by increasing the concentration. The maximum drug-resistant dose for LY8 cells is 125 ng/mL of doxorubicin and 3.5 ng/mL of vincristine, and the maximum drug-resistant dose for NU-DUL-1 cells is 25 ng/mL of doxorubicin and 0.7 ng/mL of vincristine. These cells were then treated with 2 µg/mL of 4-hydroperoxycyclophosphamide (4-HC) (Santa Cruz Biotechnology, Inc., Santa Cruz, CA) every 21 days for 3 cycles. The obtained CHO-resistant cells are referred to as LY8-CHO and NU-DUL-1-CHO, respectively. CHO-resistant cells were treated with doxorubicin, vincristine, and 4-HC every 22 days to maintain drug tolerance to CHO.

By using a similar approach, we treated LY8-R and NU-DUL-1-R cells to construct drug-resistant LY8-RCHO cells and drug-resistant NU-DUL-1-RCHO cells. To maintain RCHO resistance, LY8-RCHO cells and NU-DUL-1-RCHO cells were treated with 32 µg/mL of rituximab containing 20% (v/v) of NHS every 22 days, followed by treatment with doxorubicin, vincristine, and 4-HC. After 48 hours of treatment with doxorubicin, vincristine and 4-HC, drug resistance to CHO and RCHO was determined by a series of CCK-8 assays.

### 4. Aldefluor analysis

ALDH1 is a selectable marker for a variety of normal and cancer stem cells, including hematopoietic stem cells. Therefore, we assessed the number of cancer stem cells in hematopoietic malignancies by detecting ALDH1 positive cells. As previously described, we used an ALDEFLUOR™ kit (StemCell Technologies, Vancouver, BC, CA) to detect populations with high ALDH1 activity. Briefly, I x I06 cells were suspended in 1 mL of assay buffer containing 1µM of BAAA, a substrate of MALDH1. Suspended cells were then equally divided into two samples, one as a negative control and the other as a test sample. To form the negative control, 50 mM of DEAB, a specific ALDH inhibitor, was added to one equally-dividedsample. Cells were then incubated at 37°C for 30 minutes prior to flow cytometry analysis.

### 5. Sphere formation analysis

Cells were suspended in a serum-free medium (DMEM/F12, mixed at a ratio of 3:1) containing 0.4% (v/v) of BSA and 0.2 x B27 without vitamin A (Life Technologies, Gaithersburg, MD) and containing 10 ng/mL of recombinant EGF (PeproTech, Rocky Hill, NJ), 10 ng/mL of recombinant basic fibroblast growth factor (PeproTech, Rocky Hill, NJ), and 5µg/mL of insulin (Sigma-Aldrich, St. Louis, MO). Cells were then seeded in an ultra-low attachment 24-well plate at a density of 1 x 104 cells/mL. The medium was changed every 7 days and sphereswere counted and cells were collected at day 14. Glomus cells were subcultured at a clonal density with the above medium. Images were taken at day 14 after passage of 3 times.

### 6. CytoTox-GloTM cytotoxicity assay

Cells were seeded in a 96-well plate at a density of IxI04/100 µL/well. Cells were pretreated with IPI-145, abemaciclib, AZD4547, PF-573228, R788, or saracatinib at increasing concentrations with or without CHO for 48 hours prior to performing CytoTox-GloTM cytotoxicity assay. We performed cytotoxicity assay by using a CytoTox-Glo™ cytotoxicity assay kit (Promega Inc., Madison, WI) according to their instructions. Briefly, 50 µL of CytoTox-Glo™ cytotoxicity assay reagent was added to all wells, mixed by orbital shaking, and then incubated for 15 minutes at room temperature. Luminescence of experimental dead cells was detected with a Synergy HT microplate reader (BioTek, Biotek Winooski, MN),wherein 50 µL of lysate was added to all wells, mixed and incubated for 15 minutes at room temperature prior to detection of total luminescence. Cytotoxicity was calculated according to the following formula: Cytotoxicity (%) = Experimental dead cell luminescence/Total luminescence x 100%.

### 7. IHC staining

Tumor tissues from patients or animal models were fixed with 4% formalin, embedded in paraffin and sectioned. Paraffin sections were incubated with 3% hydrogen peroxide for 15 minutes at 37°C to block endogenous peroxidase and rinsed with 0.01M PBS followed by high pressure antigen retrieval in EDTA buffer. Sections were then incubated with rabbit anti-SOX2 monoclonal antibody (1:200; Cell Signaling Technology, Danvers, MA) overnight at 4°C. After being rinsed for 3 times in PBS, the sections were incubated with peroxidase-conjugated AffiniPure goat anti-rabbit IgG H&L (1:200; Proteintech, Chicago, IL) for 1 hour at room temperature. Immunoreactivity was then detected by using a GTVision III immunohistochemical detection kit (GK500705; Gene Tech Inc., Shanghai, China) according to its instructions. Dilution ratios of primary antibodies used for immunohistochemical staining of tissue microarrays are shown in Table 2. Quantitative scoring of immunostaining for SOX2, p-AKT, CDK6, FGFR1 and FGFR2 was performed on three independent sections under the microscope, according to the formula: score = 3 (strong positive) x percentage + 2 (medium positive) x percentage + 1 (weak positive) x percentage + 0 (negative) x percentage.

### 8. Immunoblot analysis

We performed immunoblot analysis according to a standard method and relevant antibodies are shown in Table 2.

### 9. Quantitative real-time PCR (qRT-PCR)

Total RNA from cells was extracted with a TRIzol reagent (Invitrogen, Grand Island, NY) and then transcribed into cDNA by using a reverse transcription system (Promega, Madison, WI). Input cDNA was standardized and amplified for 40 cycles on a Roche LightCycler 480 system (Roche, Basel, Switzerland) by using SYBR Green Master Mix (Invitrogen, Grand Island, NY) and gene-specific primers. We used the ACTB gene encoding β-actin as an endogenous control and samples were analyzed in triplicate. Primers for qRT-PCR are listed in Table 3.

### 10. FACS analysis

After being washed with PBS, cells were incubated with fluorescein-conjugated antibody for 30 minutes, followed by washing and resuspension of cells in PBS. Flow cytometry analysis was performed on a cytomics FC500 MPL instrument (Beckman Coulter, Inc., Brea, CA) and FlowJo software (Ashland, Inc., OR) was used for analysis. Cells were then sorted according to their relative fluorescence intensity by using a MoFlo XDP sorter (Beckman Coulter Inc., Brea, CA).

### 11. EdU cell proliferation assay

RCHO-resistant DLBCL cells were pretreated with duvelisib, abemaciclib, or AZD4547 at a concentration of 1µM for 48 or 120 hours prior to EdU assay. For 120 hours of the study, the medium was replaced with a fresh medium containing 1 µM of inhibitor at 48 hours. EdU cell proliferation assay was performed by using a cell-light EdU Apollo643 in vitro flow cytometry kit (RiboBio Inc., Guangzhou, China) according to its instructions. Next, we assayed EdU staining positive cells on a Cytomics FC500 MPL flow cytometer (Beckman Coulter, Brea, CA).

### 12. Plasmid construction and lentiviral transduction

The coding DNA sequence (CDS) of human SOX2 was obtained from a cDNA library of A549 cells by PCR amplification. This sequence was cloned into a pCDH cDNA cloning and expression lentiviral vector (cat # CD511B-1, System Biosciences, Palo Alto, CA 94303) via EcoRI and BamHI endonuclease sites to stably overexpress SOX2 in OCI-LY8 and OCI-NU-DUL-1 cells. The CDS of the firefly luciferase gene was obtained from the pGL3-Basic plasmid by PCR amplification and inserted into the pCDH cDNA cloning and expression lentiviral vector via the EcoRI and BamHI endonuclease sites. shRNA plasmids for Scramble (SCR), SOX2, ITGA1, ITGB5, CD79A and CCR7 were constructed by using a pLKO.3G cloning vector (plasmid # 14748, Addgene Inc., Cambridge, MA). 293FT cells were co-transfected with pCDH or pLKO.3G plasmids and pMD.2G and psPAX2 plasmids to generate SOX2, firefly luciferase overexpressing lentiviruses, or SOX2, ITGA1, ITGB5, CD79A, or CCR7 knockdown lentiviruses, respectively. This lentivirus was then added to a OCI-LY8, OCI-NU-DUL-1, LY8-RCHO or NU-DUL-1-RCHO medium for incubation for 48 hours. All cells transduced with lentivirus in this study were sorted by GFP with a MoFlo XDP sorter (Beckman Coulter, Brea, CA). Information regarding shRNA oligonucleotide sequences is shown in Table 3.

### 13. RNA sequencing

Total RNA was extracted from LY8-ORI, LY8-R, LY8-CHO, LY8-RCHO, NU-DUL-1, NU-DUL-1-R, NU-DUL-1-CHO, and NU-DUL-RCHO cells with a TRIzol reagent (Invitrogen, Grand Island, NY). Total RNA from each group of 3 different generations was mixed separately. RNA sequencing (RNA-seq) and bioinformatics analysis were performed by Shanghai Novelbio Co., Ltd according to its established methods. We applied the DEseq algorithm to screen differentially expressed genes, and its significance and false discovery rate (FDR) analysis followed the following criteria: (1) multiple change > 1.5 or < 0.667; (2) FDR < 0.05. Pathway analysis was used to determine significant pathways for differential genes according to the KEGG database. Series clustering analysis was performed according to the signal density of the ORI-R-RCHO and ORI-CHO-RCHO sequences to determine global trends and model profiles of expression. Fisher's exact test and multiple comparison test were used to select significant pathways, with significance thresholds defined by P-value and FDR. We screened enrichment pathways with a significant increasing trend in the ORI-R-RCHO and ORI-CHO-RCHO sequences and then analyzed the interaction frequencies of these pathways between these groups by using Cytoscape software.

RNA-seq data for this study is available in the NCBI GEO database through GEO series accession numbers GSE112989 and GSE113001.

### 14. Gene set enrichment analysis

We performed RNA-seq-based gene set enrichment analysis (GSEA) of differentially expressed gene function by using the GSEA software of the Broad Institute (Massachusetts Institute of Technology). A pre-ordered version of this software was used to determine significantly enriched pathways, and enriched pathways with FDR < 0.25 were considered significant. The PI3K-AKT signaling pathway gene set used in this study consisted of 341 genes of the PI3K-AKT signaling pathway SuperPath in the PathCards pathway unified database (version 4.6.0.37, Weizmann Institute of Science). The GSEA term KEGG_CHEMOKINE_SIGNALING_PATHWAY is also used to recognize chemokine signaling pathways.

### 15. Statistical method

Unless otherwise stated, data in the embodiments of the present invention are expressed as mean ± standard deviation. Data were unpaired by using two-tailed Student's t-test to determine significant differences between the two groups, and P<0.05 was considered statistically significant. For IHC staining scores of tissue microarrays, significance was determined by two-tailed paired t-test, and P<0.05 was considered statistically significant. For the total photon flux of the animal model, significance was determined by one-tailed Mann-Whitney test, and P<0.05 was considered statistically significant. We applied the Mantel-Cox test to compare survival of two groups of xenograft models, with P<0.05 considered statistically significant.

**Table 2. List of antibody commodity used in the embodiments**

| **Anti body** | **Producer** | **Applications in embodiments** | **Cargo number** |
|---|---|---|---|
| CD34 [ICO-115] | abcam | WB (1: 1,000) | ab187282 |
| CD133 | HuaBio Co., Ltd. | WB (1: 1,000) | R121101 |
| β-actin (C4) | Santa Cruz Biotechnology | WB (1: 1,000) | sc-47778 |
| Sox2 (D6D9) XP Rabbit mAb | Cell Signaling Technology | WB (1: 1,000), IHC (1: 200) | 3579 |
| OCT4 [EPR17929] | abcam | WB (1: 1,000) | ab181557 |
| Nanog [EPR2027(2)] | abcam | WB (1: 1,000) | ab109250 |
| KLF4 [EPR19590] | abcam | WB (1: 1,000) | ab215036 |
| C-Myc (D3N8F) Rabbit mAb | Cell Signaling Technology | WB (1: 1,000) | 13987 |
| Phospho-Akt (Ser473) (D9E) | Cell Signaling Technology | IHC (1: 100) WB (1: 1,000) | 4060 |
| PI3 Kinase p110α (C73F8) | Cell Signaling Technology | WB (1: 1,000) | 4249 |
| PI3 Kinase p110β (C33D4) | Cell Signaling Technology | WB (1: 1,000) | 3011 |
| PI3 Kinase p110γ (D55D5) | Cell Signaling Technology | WB (1: 1,000) | 5405 |
| PI3 Kinase p110δ (D1Q7R) | Cell Signaling Technology | WB (1: 1,000) | 34050 |
| PI3 Kinase p85 (19H8) | Cell Signaling Technology | WB (1: 1,000) | 4257 |
| Akt1 (C73H10) | Cell Signaling Technology | WB (1: 1,000) | 2938 |
| Ubiquitin (P4D1) | Cell Signaling Technology | WB (1: 1,000) | 3936 |
| Phospho-FAK (Tyr397) (D20B1) | Cell Signaling Technology | WB (1: 1,000) | 8556 |
| FAK (D2R2E) | Cell Signaling Technology | WB (1: 1,000) | 13009 |
| Integrin 1 (A-9) | Santa Cruz Biotechnology | WB (1: 500) | sc-271034 |
| Integrin beta 5 | abcam | WB (1: 1,000) | ab15459 |
| Phospho-Aktl (Ser473) (D7F10) | Cell Signaling Technology | WB (1: 1,000) | 9018 |
| CD79a [EPR3619] | abcam | WB (1: 1,000) | ab133483 |
| P-Lyn (Y 396) | abcam | WB (1: 1,000) | ab226778 |
| Lyn (C13F9) | Cell Signaling Technology | WB (1: 1,000) | 2796 |
| SHP-1 [EPR5519] | abcam | WB (1: 1,000) | ab124942 |
| Phospho-SHP-1 (Tyr564) (D11G5) | Cell Signaling Technology | WB (1: 1,000) | 8849 |
| TCF3/TCF7L1 (D15G11) | Cell Signaling Technology | WB (1: 1,000) | 2883 |
| Phospho-Syk (Tyr525/526) (C87C1) | Cell Signaling Technology | WB (1: 1,000) | 2710 |
| Syk (N-19) | Santa Cruz Biotechnology | WB (1: 500) | sc-1077 |
| CCR7 [E75] | abcam | WB (1: 500) | ab32075 |
| p-Src (Y418) [EP503Y] | abcam | WB (1: 1,000) | ab40660 |
| Src [EPR5496] | abcam | WB (1: 1,000) | ab109381 |
| CDK6 [EPR4515] | abcam | WB (1: 1,000) IHC (1: 100) | ab124821 |
| FGFR1 | abcam | WB (1: 1,000) IHC (1: 400) | ab10646 |
| FGFR2 (C17) | Santa Cruz Biotechnology | WB (1: 1,000) IHC (1: 200) | sc-122 |
| CD20 (L26) | Santa Cruz Biotechnology | WB (1: 1,000) | sc-58985 |
| PE anti-human CD55 (IA10) | BD Pharmingen | FACS (20 L/test) | 555694 |
| PE anti-Human CD59 (H19) | BioLegend | FACS (5 L/test) | 304708 |
| APC anti-human CD46 (TRA-2-10) | BioLegend | FACS (5 L/test) | 352405 |
| APC anti-human CD20 (2H7) | BioLegend | FACS (5 L/test) | 302310 |
| Goat anti-mouse IgG-HRP | Santa Cruz Biotechnology | WB (1: 10,000) | sc-2005 |
| Goat anti-rabbit IgG-HRP | Santa Cruz Biotechnology | WB (1: 10,000) | sc-2004 |
| Peroxidase-conjugated affinity good anti-rabbit IgG H&L | Proteintech Group Inc. | IHC (1: 200) | SA00001-2 |
| Normal mouse IgG | Santa Cruz Biotechnology | 2 g/time | sc-2025 |

**Table 3. List of primers and shRNA sequences used in the embodiments**

| **Primer** | **5 'to 3'** |
|---|---|
| *SOX2* qRT-PCR forward primer | GCCGAGTGGAAACTTTTGTCG |
| *SOX2* qRT-PCR reverse primer | GGCAGCGTGTACTTATCCTTCT |
| *ITGA1* qRT-PCR forward primer | CGCTGCTGCGTATCATTCAA |
| *ITGA1* qRT-PCR reverse primer | GGCCAACTAACGGAGAACCA |
| *ITGA2* qRT-PCR forward primer | AGTGGCTTTCCTGAGAACCG |
| *ITGA2* qRT-PCR reverse primer | GATCAAGCCGAGGCTCATGT |
| *ITGA2B* qRT-PCR forward primer | AACACCCTGAGCCGCATTTA |
| *ITGA2B* qRT-PCR reverse primer | AATAATAGCCGCCAGGAGCC |
| *ITGA3* qRT-PCR forward primer | CCCTATTCCTCCGAACCAGC |
| *ITGA3* qRT-PCR reverse primer | CGCCTTCTGCCTCTTAGCTT |
| *ITGA4* qRT-PCR forward primer | CAACGAAAGAACTGGACGGC |
| *ITGA4* qRT-PCR reverse primer | TAGAAGCGGAGCTGTGTGAC |
| *ITGA5* qRT-PCR forward primer | ACATCTGTGTGCCTGACCTG |
| *ITGA5* qRT-PCR reverse primer | CTCACCCACATTCTGGGCAT |
| *ITGA6* qRT-PCR forward primer | TTCGGGAGTACCTTGGTGGA |
| *ITGA6* qRT-PCR reverse primer | AGAGCGTTTAAAGAATCCACACT |
| *ITGA7* qRT-PCR forward primer | ATTTCCCTTGCATTCGCTGG |
| *ITGA7* qRT-PCR reverse primer | CGTCCAGATTGAAGGCGACA |
| *ITGA8* qRT-PCR forward primer | CTTATTGTGGGAGGACCTGGG |
| *ITGA8* qRT-PCR reverse primer | GTAAACTCCCCAGCAGCAACT |
| *ITGA9* qRT-PCR forward primer | GGCTGTGTTTAAGTGCCGTG |
| *ITGA9* qRT-PCR reverse primer | ATCCACTCATCATCGCGGTC |
| *ITGA10* qRT-PCR forward primer | GATCAGGCATGGAACTCCCC |
| *ITGA10* qRT-PCR reverse primer | ACAGGGCAGCGATAAACGTC |
| *ITGA11* qRT-PCR forward primer | TGGCCAGGGTTCACGGAC |
| *ITGA11* qRT-PCR reverse primer | CCGTGGATCACTGGACACTT |
| *ITGAD* qRT-PCR forward primer | CATGAGATTCAGCCCTGTGGA |
| *ITGAD* qRT-PCR reverse primer | TCCAGTGCCAGATCAAACCT |
| *ITGAE* qRT-PCR forward primer | CTCAAGTGGGGAGTGTCACC |
| *ITGAE* qRT-PCR reverse primer | CAACACCCTGCATTTTGGGG |
| *ITGAL* qRT-PCR forward primer | CTGTAAGAGGCCAAAGGGCA |
| *ITGAL* qRT-PCR reverse primer | GCGCGAAGAAAAAGAACCCA |
| *ITGAM* qRT-PCR forward primer | GCCAGGACCTCACAATGGAT |
| *ITGAM* qRT-PCR reverse primer | CTTGCCACTTCCCTGGGATT |
| *ITGAV* qRT-PCR forward primer | TCCCATCAGTGGTTTGGAGC |
| *ITGAV* qRT-PCR reverse primer | TGATCTACATGGAGCATACTCAACA |
| *ITGAX* qRT-PCR forward primer | TGGCTTCTTCAAGCGTCAGT |
| *ITGAX* qRT-PCR reverse primer | GAGGGTAATGGGGAGTGGGC |
| *ITGB1* qRT-PCR forward primer | GCCGCGCGGAAAAGATGAAT |
| *ITGB1* qRT-PCR reverse primer | GAATTTGTGCACCACCCACAA |
| *ITGB2* qRT-PCR forward primer | GTGTCAGGACTTTACGACCCG |
| *ITGB2* qRT-PCR reverse primer | ACTCCTGAGAGAGGACGCA |
| *ITGB3* qRT-PCR forward primer | ACCAGTAACCTGCGGATTGG |
| *ITGB3* qRT-PCR reverse primer | CTCATTGAAGCGGGTCACCT |
| *ITGB4* qRT-PCR forward primer | AAGGGCAACATCCATCTGAAAC |
| *ITGB4* qRT-PCR reverse primer | GCTGACCGCACCTCTTTTTG |
| *ITGB5* qRT-PCR forward primer | ATACCTGGAACAACGGTGGAG |
| *ITGB5* qRT-PCR reverse primer | GGCTGATCCCAGACTGACAA |
| *ITGB6* qRT-PCR forward primer | ATCGGTCTGCACAGCAAGAA |
| *ITGB6* qRT-PCR reverse primer | CAGGCACACTGAGGTCCAAT |
| *ITGB7* qRT-PCR forward primer | GGCTCTTCTACCACTACGGC |
| *ITGB7* qRT-PCR reverse primer | CATTCTGTGGCATCCCCTGT |
| *ITGB8* qRT-PCR forward primer | AGCTGCAACTAATGGTGTTGG |
| *ITGBB* qRT-PCR reverse primer | AACCCAAACAAAGCCCGAAG |
| CCR7qRT-PCR forward primer | ATGCCTGTGTCAAGATGAGGTCA |
| CCR7qRT-PCR reverse primer | AAGTTCCGCACGTCCTTCTT |
| *CD79A* qRT-PCR forward primer | AGAACGAGAAGCTCGGGTTG |
| *CD79A* qRT-PCR reverse primer | CCCCGGGAGATGTCCTCATA |
| *CD20* qRT-PCR forward primer | AACTCAGCAGTAGGCCTTGC |
| *CD20* qRT-PCR reverse primer | GCAGTCTTACCTTGTGTCATGC |
| *Firefly Luciferase* CDS forward primer | ATGGAAGACGCCAAAAACATAAAG |
| *Firefly Luciferase* CDS reverse primer | TTACACGGCGATCTTTCCGCCCTT |
| *SOX2* shRNA#1 forward primer | AATTAGGAGCACCCGGATTATAAATCTC GAGATTTATAATCCGGGTGCTCCTTTTTTAT |
| *SOX2* shRNA#1 reverse primer | AAAAAAAAGGAGCACCCGGATTATAAAT CTCGAGATTTATAATCC GGGTGCTCCT |
| *SOX2* shRNA#2 forward primer | AATTGGTTGACACCGTTGGTAATTTCTCG AGAAATACCAACGGTGTCAACCTTTTTTTAT |
| *SOX2* shRNA#2 reverse primer | AAAAAAAGGTTGACACCGTTGGTAATTTC TCGAGAAATTACCAACGGTGTCAACC |
| *ITGA1* shRNA#1 forward primer | AATTTCTGGAGATGTGCTCTATATTCTCG AGAATATAGAGCACATCTCCAGATTTTTTTAT |
| *ITGA1* shRNA#1 reverse primer | AAAAAAATCTGGAGATGTGCTCTATATTC TCGAGAATATAGAGCACATCTCCAGA |
| *ITGA1* shRNA#2 forward primer | AATTACTCTGGGAGAAAGAATATTTCTCG AGAAATATTCTTTCTCCCAGAGTTTTTTTAT |
| *ITGA1* sh RNA#2 reverse primer | AAAAAAAACTCTGGGAGAAAGAATATTTC TCGAGAAATATTCTTTCTCCCAGAGT |
| *ITGB5* shRNA#1 forward primer | AATTGGATTGGAAGTAAAGATTAAACTCG AGTTTAATCTTTACTTCCAATCCTTTTTTTAT |
| *ITGB5* shRNA#1 reverse primer | AAAAAAAGGATTGGAAGTAAAGATTAAAC TCGAGTTTAATCTTTACTTCCAATCC |
| *ITGB5* shRNA#2 forward primer | AATTTTCCTTCATCTTGTGTAAATACTCG AGTATTTACACAAGATGAAGGAATTTTTTTAT |
| *ITGB5* shRNA#2 reverse primer | AAAAAAATTCCTTCATCTTGTGTAAATAC TCGAGTATTTACACAAGATGAAGGAA |
| *CD79A* shRNA#1 forward primer | AATTACTTCCAATGCCCGCACAATACTCG AGTATTGTGCGGGCATTGGAAGTTTTTTAT |
| *CD79A* shRNA#1 reverse primer | AAAAAAAACTTCCAATGCCCGCACAATAC TCGAGTATTGTGCGGGCATTGGAAGT |
| *CD79A* shRNA#2 forward primer | AATTCAGGGCCACTTAGTGATAATACTC GAGTATTATCACTAAGTGGCCCTGTTTTTTTA T |
| *CD79A* shRNA#2 reverse primer | AAAAAAACAGGGCCACTTAGTGATAATA CTCGAGTATTATCACTAAGTGGCCCTG |
| *CCR7* shRNA#1 forward primer | AATTCGAGCTTGTTCTTTGTTCTTTCTCG AGAAAGAACAAAGAACAAGCTCGTTTTTTTAT |
| *CCR7* shRNA#1 reverse primer | AAAAAAACGAGCTTGTTCTTTGTTCTTTC TCGAGAAAGAACAAAGAACAAGCTCG |
| *CCR7* shRNA#2 forward primer | AATTCCCTTTCTTGTACGCCTTCATCTCG AGATGAAGGCGTACAAGAAAGGGTTTTTTTAT |
| CCR7shRNA#2 reverse primer | AAAAAAACCCTTTCTTGTACGCCTTCATC TCGAGATGAAGGCGTACAAGAAAGGG |
| scramble shRNA forward primer | AATTCCTAAGGTTAAGTCGCCCTCGCTC GAGCGAGGGCGACTTAACCTTAGGTTTTTTT |
| scramble shRNA reverse primer | AAAAAAACCTAAGGTTAAGTCGCCCTCG CTCGAGCGAGGGCGACTTAACCTTAGG |

Preferred embodiments of the invention are described in detail above. It should be understood that numerous modifications and variations can be made by those of ordinary skill in the art without inventive labor in accordance with the concept of the invention. Therefore, all technical solutions that can be obtained by those skilled in the art through logical analysis, reasoning or limited experiments on the basis of the prior art according to the concept of the invention shall be within the protection scope determined by the claims.

<120>   一种治疗 B 细胞淋巴瘤的药物组合物

<130>   ZLYY239-181015A

<141>   2018-07-03

<160>   84

## Claims

1. A pharmaceutical composition for treating B-cell lymphoma, comprising a PI3K/AKT signaling pathway inhibitor and a chemotherapeutic drug.

2. The pharmaceutical composition according to claim 1, further comprising a monoclonal antibody targeting CD20.

3. The pharmaceutical composition according to claim 2, **characterized in that** the monoclonal antibody targeting CD20 is rituximab.

4. The pharmaceutical composition according to claim 1 or 2, further comprising a pharmaceutically acceptable carrier or excipient.

5. The pharmaceutical composition according to claim 1 or 2, **characterized in that** the chemotherapeutic drug is selected from one or more of cyclophosphamide, doxorubicin and vincristine.

6. The pharmaceutical composition according to claim 1 or 2, **characterized in that** the PI3K/AKT signaling pathway inhibitor is selected from PI3K inhibitors, AKT inhibitors or inhibitors of related proteins upstream and downstream of PI3K/AKT.

7. The pharmaceutical composition according to claim 1 or 2, **characterized in that** the PI3K/AKT signaling pathway inhibitor is selected from duvelisib, a derivative thereof, a pharmaceutically acceptable salt thereof, a solvate thereof, and a prodrug thereof.

8. The pharmaceutical composition according to claim 1 or 2, **characterized in that** the PI3K/AKT signaling pathway inhibitor is selected from FAK inhibitors, Syk inhibitors and Src inhibitors.

9. The pharmaceutical composition according to claim 1 or 2, **characterized in that** the PI3K/AKT signaling pathway inhibitor is selected from abemaciclib, a derivative thereof, a pharmaceutically acceptable salt thereof, a solvate thereof, and a prodrug thereof.

10. The pharmaceutical composition according to claim 1 or 2, **characterized in that** the PI3K/AKT signaling pathway inhibitor is selected from AZD4547, a derivative thereof, a pharmaceutically acceptable salt thereof, a solvate thereof, and a prodrug thereof.

11. A pharmaceutical composition for treating B-cell lymphoma, comprising a first preparation formed by a PI3K/AKT signaling pathway inhibitor and a pharmaceutically acceptable carrier, a second preparation formed by a monoclonal antibody targeting CD20 and a pharmaceutically acceptable carrier, and a third preparation formed by a chemotherapeutic drug and a pharmaceutically acceptable carrier.

12. The pharmaceutical composition according to claim 11, **characterized in that** the PI3K/AKT signaling pathway inhibitor is selected from duvelisib, a derivative thereof, a pharmaceutically acceptable salt thereof, a solvate thereof, and a prodrug thereof.

13. Use of a PI3K/AKT signaling pathway inhibitor, a monoclonal antibody targeting CD20 and a chemotherapeutic drug in the preparation of a combined drug for the treatment of B-cell lymphoma.

14. The use according to claim 13, **characterized in that** the B-cell lymphoma is diffuse large B-cell lymphoma.

15. The use according to claim 13, **characterized in that** the B-cell lymphoma is B-cell lymphoma resistant to chemotherapeutic drugs.

16. The use according to claim 13, **characterized in that** the B-cell lymphoma is B-cell lymphoma with an elevated proportion of CSCs.


<170>   SIPOSequenceListing 1.0

<210>   1
<211>   21
<212>   DNA
<213>   Artificial Sequence

<400>   1
gccgagtgga aacttttgtc g                                                21

<210>   3
<211>   22
<212>   DNA
<213>   Artificial Sequence

<400>   3
ggcagcgtgt acttatcctt ct                                               22

<210>   3
<211>   20
<212>   DNA
<213>   Artificial Sequence

<400>   3
cgctgctgcg tatcattcaa                                                  20

<210>   4
<211>   20
<212>   DNA
<213>   Artificial Sequence

<400>   4
ggccaactaa cggagaacca                                                  20

<210>   5
<211>   20
<212>   DNA
<213>   Artificial Sequence

<400>   5
agtggctttc ctgagaaccg                                                  20

<210>   6
<211>   20
<212>   DNA
<213>   Artificial Sequence

<400>   6

gatcaagccg aggctcatgt                                                    20

<210>  7
<211>  20
<212>  DNA
<213>  Artificial Sequence

<400>  7
aacaccctga gccgcattta                                                    20

<210>  8
<211>  20
<212>  DNA
<213>  Artificial Sequence

<400>  8
aataatagcc gccaggagcc                                                    20

<210>  9
<211>  20
<212>  DNA
<213>  Artificial Sequence

<400>  9
ccctattcct ccgaaccagc                                                    20

<210>  10
<211>  20
<212>  DNA
<213>  Artificial Sequence

<400>  10
cgccttctgc ctcttagctt                                                    20

<210>  11
<211>  20
<212>  DNA
<213>  Artificial Sequence

<400>  11
caacgaaaga actggacggc                                                    20

<210>  12
<211>  20
<212>  DNA
<213>  Artificial Sequence

<400>  12
tagaagcgga gctgtgtgac                                                    20

<210>  13
<211>  20
<212>  DNA
<213>  Artificial Sequence

<400>  13
acatctgtgt gcctgacctg                                                    20

<210>  14
<211>  20
<212>  DNA
<213>  Artificial Sequence

<400>  14
ctcacccaca ttctgggcat                                                   20

<210>  15
<211>  20
<212>  DNA
<213>  Artificial Sequence

<400>  15
ttcgggagta ccttggtgga                                                   20

<210>  16
<211>  23
<212>  DNA
<213>  Artificial Sequence

<400>  16
agagcgttta aagaatccac act                                               23

<210>  17
<211>  20
<212>  DNA
<213>  Artificial Sequence

<400>  17
atttcccttg cattcgctgg                                                   20

<210>  18
<211>  20
<212>  DNA
<213>  Artificial Sequence

<400>  18
cgtccagatt gaaggcgaca                                                   20

<210>  19
<211>  21
<212>  DNA
<213>  Artificial Sequence

<400>  19
cttattgtgg gaggacctgg g                                                 21

<210>  20
<211>  21
<212>  DNA
<213>  Artificial Sequence

<400>  20
gtaaactccc cagcagcaac t                                                 21

<210>  21
<211>  20

<212>  DNA
<213>  Artificial Sequence

<400>  21
ggctgtgttt aagtgccgtg                                                    20

<210>  22
<211>  20
<212>  DNA
<213>  Artificial Sequence

<400>  22
atccactcat catcgcggtc                                                    20

<210>  23
<211>  20
<212>  DNA
<213>  Artificial Sequence

<400>  23
gatcaggcat ggaactcccc                                                    20

<210>  24
<211>  20
<212>  DNA
<213>  Artificial Sequence

<400>  24
acagggcagc gataaacgtc                                                    20

<210>  25
<211>  18
<212>  DNA
<213>  Artificial Sequence

<400>  25
tggccagggt tcacggac                                                      18

<210>  26
<211>  20
<212>  DNA
<213>  Artificial Sequence

<400>  26
ccgtggatca ctggacactt                                                    20

<210>  27
<211>  21
<212>  DNA
<213>  Artificial Sequence

<400>  27
catgagattc agccctgtgg a                                                  21

<210>  28
<211>  20
<212>  DNA
<213>  Artificial Sequence

<400>  28
tccagtgcca gatcaaacct                                                   20

<210>  29
<211>  20
<212>  DNA
<213>  Artificial Sequence

<400>  29
ctcaagtggg gagtgtcacc                                                   20

<210>  30
<211>  20
<212>  DNA
<213>  Artificial Sequence

<400>  30
caacaccctg cattttgggg                                                   20

<210>  31
<211>  20
<212>  DNA
<213>  Artificial Sequence

<400>  31
ctgtaagagg ccaaagggca                                                   20

<210>  32
<211>  20
<212>  DNA
<213>  Artificial Sequence

<400>  32
gcgcgaagaa aaagaaccca                                                   20

<210>  33
<211>  20
<212>  DNA
<213>  Artificial Sequence

<400>  33
gccaggacct cacaatggat                                                   20

<210>  34
<211>  20
<212>  DNA
<213>  Artificial Sequence

<400>  34
cttgccactt ccctgggatt                                                   20

<210>  35
<211>  20
<212>  DNA
<213>  Artificial Sequence

<400>  35

tcccatcagt ggtttggagc                                                    20

<210>  36
<211>  25
<212>  DNA
<213>  Artificial Sequence

<400>  36
tgatctacat ggagcatact caaca                                              25

<210>  37
<211>  20
<212>  DNA
<213>  Artificial Sequence

<400>  37
tggcttcttc aagcgtcagt                                                    20

<210>  38
<211>  20
<212>  DNA
<213>  Artificial Sequence

<400>  38
gagggtaatg gggagtgggc                                                    20

<210>  39
<211>  20
<212>  DNA
<213>  Artificial Sequence

<400>  39
gccgcgcgga aaagatgaat                                                    20

<210>  40
<211>  21
<212>  DNA
<213>  Artificial Sequence

<400>  40
gaatttgtgc accacccaca a                                                  21

<210>  41
<211>  21
<212>  DNA
<213>  Artificial Sequence

<400>  41
gtgtcaggac tttacgaccc g                                                  21

<210>  42
<211>  19
<212>  DNA
<213>  Artificial Sequence

<400>  42
actcctgaga gaggacgca                                                     19

<210>  43
<211>  20
<212>  DNA
<213>  Artificial Sequence

<400>  43
accagtaacc tgcggattgg                                                    20

<210>  44
<211>  20
<212>  DNA
<213>  Artificial Sequence

<400>  44
ctcattgaag cgggtcacct                                                    20

<210>  45
<211>  22
<212>  DNA
<213>  Artificial Sequence

<400>  45
aagggcaaca tccatctgaa ac                                                 22

<210>  46
<211>  20
<212>  DNA
<213>  Artificial Sequence

<400>  46
gctgaccgca cctcttttg                                                     20

<210>  47
<211>  21
<212>  DNA
<213>  Artificial Sequence

<400>  47
atacctggaa caacggtgga g                                                  21

<210>  48
<211>  20
<212>  DNA
<213>  Artificial Sequence

<400>  48
ggctgatccc agactgacaa                                                    20

<210>  49
<211>  20
<212>  DNA
<213>  Artificial Sequence

<400>  49
atcggtctgc acagcaagaa                                                    20

<210>  50
<211>  20

<212>  DNA
<213>  Artificial Sequence

<400>  50
caggcacact gaggtccaat                                                   20

<210>  51
<211>  20
<212>  DNA
<213>  Artificial Sequence

<400>  51
ggctcttcta ccactacggc                                                   20

<210>  52
<211>  20
<212>  DNA
<213>  Artificial Sequence

<400>  52
cattctgtgg catcccctgt                                                   20

<210>  53
<211>  21
<212>  DNA
<213>  Artificial Sequence

<400>  53
agctgcaact aatggtgttg g                                                 21

<210>  54
<211>  20
<212>  DNA
<213>  Artificial Sequence

<400>  54
aacccaaaca aagcccgaag                                                   20

<210>  55
<211>  23
<212>  DNA
<213>  Artificial Sequence

<400>  55
atgcctgtgt caagatgagg tca                                               23

<210>  56
<211>  20
<212>  DNA
<213>  Artificial Sequence

<400>  56
aagttccgca cgtccttctt                                                   20

<210>  57
<211>  20
<212>  DNA
<213>  Artificial Sequence

<400>  57
agaacgagaa gctcgggttg                                                   20

<210>  58
<211>  20
<212>  DNA
<213>  Artificial Sequence

<400>  58
ccccgggaga tgtcctcata                                                   20

<210>  59
<211>  20
<212>  DNA
<213>  Artificial Sequence

<400>  59
aactcagcag taggccttgc                                                   20

<210>  60
<211>  22
<212>  DNA
<213>  Artificial Sequence

<400>  60
gcagtcttac cttgtgtcat gc                                                22

<210>  61
<211>  24
<212>  DNA
<213>  Artificial Sequence

<400>  61
atggaagacg ccaaaaacat aaag                                              24

<210>  62
<211>  24
<212>  DNA
<213>  Artificial Sequence

<400>  62
ttacacggcg atctttccgc cctt                                              24

<210>  63
<211>  61
<212>  DNA
<213>  Artificial Sequence

<400>  63
   aattaggagc acccggatta taaatctcga gatttataat ccgggtgctc ctttttttta       60
   t                                                                       61

<210>  64
<211>  55
<212>  DNA
<213>  Artificial Sequence

<400>  64
aaaaaaaagg agcacccgga ttataaatct cgagatttat aatccgggtg ctcct          55

<210>  65
<211>  60
<212>  DNA
<213>  Artificial Sequence

<400>  65
aattggttga caccgttggt aatttctcga gaaataccaa cggtgtcaac cttttttat     60

<210>  66
<211>  55
<212>  DNA
<213>  Artificial Sequence

<400>  66
aaaaaaaggt tgacaccgtt ggtaatttct cgagaaatta ccaacggtgt caacc          55

<210>  67
<211>  61
<212>  DNA
<213>  Artificial Sequence

<400>  67
   aatttctgga gatgtgctct atattctcga gaatatagag cacatctcca gattttttta     60
   t                                                                     61

<210>  68
<211>  55
<212>  DNA
<213>  Artificial Sequence

<400>  68
aaaaaaatct ggagatgtgc tctatattct cgagaatata gagcacatct ccaga          55

<210>  69
<211>  61
<212>  DNA
<213>  Artificial Sequence

<400>  69
aattactctg ggagaaagaa tatttctcga gaaatattct ttctcccaga gtttttttta     60
t                                                                     61

<210>  70
<211>  55
<212>  DNA
<213>  Artificial Sequence

<400>  70
aaaaaaaact ctgggagaaa gaatatttct cgagaaatat tctttctccc agagt          55

<210>  71
<211>  61
<212>  DNA
<213>  Artificial Sequence

<400>  71
   aattggattg gaagtaaaga ttaaactcga gtttaatctt tacttccaat ccttttttta      60
   t                                                                     61

<210>  72
<211>  55
<212>  DNA
<213>  Artificial Sequence

<400>  72
aaaaaaagga ttggaagtaa agattaaact cgagtttaat ctttacttcc aatcc          55

<210>  73
<211>  61
<212>  DNA
<213>  Artificial Sequence

<400>  73
aattttcctt catcttgtgt aaatactcga gtatttacac aagatgaagg aattttttta      60
t                                                                     61

<210>  74
<211>  55
<212>  DNA
<213>  Artificial Sequence

<400>  74
aaaaaaattc cttcatcttg tgtaaatact cgagtattta cacaagatga aggaa          55

<210>  75
<211>  61
<212>  DNA
<213>  Artificial Sequence

<400>  75
aattacttcc aatgcccgca caatactcga gtattgtgcg ggcattggaa gtttttttta      60
t                                                                     61

<210>  76
<211>  55
<212>  DNA
<213>  Artificial Sequence

<400>  76
aaaaaaaact tccaatgccc gcacaatact cgagtattgt gcgggcattg gaagt          55

<210>  77
<211>  61
<212>  DNA
<213>  Artificial Sequence

<400>  77
aattcagggc cacttagtga taatactcga gtattatcac taagtggccc tgttttttta      60
t                                                                     61

<210>  78
<211>  55
<212>  DNA

<213>  Artificial Sequence

<400>  78
aaaaaaacag ggccacttag tgataatact cgagtattat cactaagtgg ccctg          55

<210>  79
<211>  61
<212>  DNA
<213>  Artificial Sequence

<400>  79
aattcgagct tgttctttgt tctttctcga gaaagaacaa agaacaagct cgttttttta     60
t                                                                     61

<210>  80
<211>  55
<212>  DNA
<213>  Artificial Sequence

<400>  80
aaaaaaacga gcttgttctt tgttctttct cgagaaagaa caaagaacaa gctcg          55

<210>  81
<211>  61
<212>  DNA
<213>  Artificial Sequence

<400>  81
aattcccttt cttgtacgcc ttcatctcga gatgaaggcg tacaagaaag ggttttttta     60
t                                                                     61

<210>  82
<211>  55
<212>  DNA
<213>  Artificial Sequence

<400>  82
aaaaaaaccc tttcttgtac gccttcatct cgagatgaag gcgtacaaga aaggg          55

<210>  83
<211>  59
<212>  DNA
<213>  Artificial Sequence

<400>  83
aattcctaag gttaagtcgc cctcgctcga gcgagggcga cttaacctta ggttttttt      59

<210>  84
<211>  55
<212>  DNA
<213>  Artificial Sequence

<400>  84
aaaaaaacct aaggttaagt cgccctcgct cgagcgaggg cgacttaacc ttagg          55